# EUROPEAN PATENT APPLICATION

(11) **EP 3 722 427 A1**
(43) Date of publication of application: **14.10.2020**
(21) Application number: 19735887.2
(22) Date of filing: 07.01.2019
(51) Int. Cl.: C12N 15/67, C12N 15/23, C12N 15/24, C12N 15/26, C12N 15/27, C12N 15/28, A61K 48/00

(54) **METHOD AND SYSTEM FOR SCREENING ANTI-TUMOUR SUBSTANCES**

(30) Priority: 08.01.2018 CN 201810013834
(71) Applicant: Zhang, Jinyu, Chongqing 400037 (CN)
(72) Inventor: Zhang, Jinyu, Chongqing 400037 (CN)
(74) Representative: Böhm, Brigitte
(86) International application number: PCT/CN2019/070576
(87) International publication number: WO 2019/134692

(57) **Abstract**

Provided is a system for screening anti-tumour substances. The system comprises a first expression vector which encodes a first regulatory factor and a second expression vector which encodes a biological macromolecule, and the expression of the biological macromolecule can be co-regulated by the first regulatory factor and an inducer. Also provided are a method for preparing the system, a method for screening anti-tumor substances by using the system and anti-tumour substances obtained by screening.

## Description

### Technical Field

The present application relates to the biomedical field, in particular to a system and a method for screening anti-tumor substances.

### Background

In conventional anti-tumor drug screening, researchers usually add various drugs directly to the cultured tumor cells, and analyze the effects of the drugs on the proliferation, differentiation or other tumorigenesis-related biological activities of the tumor cells by various means, so as to find the drugs that can directly affect tumor cells. This method has a very important limitation, that is, it takes the effect of the drugs on the tumor cells themselves as the first evaluation factor, but avoids and overlooks the effects of the complex microenvironment of tumors on tumorigenesis. As a result, the screened drugs cannot obtain the inhibitory effect *in vivo* as shown in the cultured cells in vitro.

It can be seen that the significant differences between tumors *in vitro* and *in vivo* result in that a large number of *in vitro* screened anti-tumor drugs cannot be effective *in vivo.* More importantly, as in the current common *in vivo* anti-tumor drug evaluation models, nude mice are inoculated with tumor cells and then subjected to drug intervention. Due to the lack of a normal immune system in nude mice, the tumor microenvironment of the tumors inoculated in nude are far away from that of the tumors of normal mice, and the evaluation results obtained are not very good for reference. Meanwhile, a combinational therapy of tumor drugs has recently become a trend in recent years, but currently no reliable *in vivo* screening method for screening the combinations of anti-tumor drugs is available.

### Summary of the Invention

The present application provides a system and a method for screening anti-tumor substances (in particular, a combination of anti-tumor substances). The system and/or method has one or more of the following properties: 1) simulating and reproducing the complex microenvironments in which tumor occurs; 2) adapting better to a subject having an immune system or immunity; 3) screened anti-tumor substances having an effective inhibition effect on *in vivo* tumors; 4) effectively obtaining a combination of multiple anti-tumor substances having a synergistic effect; and 5) simplicity of constructing method and simplicity and easiness of using method.

In one aspect, the present application provides a system for controlled expression of a biological macromolecule, comprising: 1) a first expression vector encoding a first regulatory factor; 2) a second expression vector encoding a biological macromolecule, wherein an expression of the biological macromolecule can be co-regulated by the first regulatory factor expressed by the first expression vector and an inducer, and the biological macromolecule is a candidate anti-tumor substance to be screened.

In another aspect, the present application provides a system for screening anti-tumor substances, comprising: 1) a first expression vector encoding a first regulatory factor; 2) a second expression vector encoding a biological macromolecule, wherein an expression of the biological macromolecule can be co-regulated by the first regulatory factor expressed by the first expression vector and an inducer.

In certain embodiments, the first regulatory factor comprises a transcription factor.

In certain embodiments, the inducer changes the conformation of the first regulatory factor.

In certain embodiments, the inducer changes the conformation of the first regulatory factor by binding to the first regulatory factor.

In certain embodiments, the inducer binds to the regulatory factor to form a transcription complex, and the transcription complex activates the expression of the biological macromolecule encoded by the second expression vector.

In certain embodiments, the first expression vector and the second expression vector are different expression vectors independent of each other.

In certain embodiments, the system comprises two or more types of second expression vectors, and the biological macromolecule encoded by each type of second expression vectors is different from the biological macromolecule encoded by at least another type of second expression vectors in the system.

In certain embodiments, the system comprises a cell comprising the first expression vector and/or the second expression vector.

In certain embodiments, the system comprises two or more types of cells, each type of cells comprises the first expression vector and the second expression vector, and the biological macromolecule encoded by the second expression vector in each type of cells is different from the biological macromolecule encoded by the second expression vector in at least another type of cells.

In certain embodiments, the first expression vector and/or the second expression vector are integrated into the genome of the cells.

In certain embodiments, the cell comprises a tumor cell. In certain embodiments, the tumor cell comprises a melanoma cell. In certain embodiments, the melanoma cell comprises a mouse melanoma cell B16.

In certain embodiments, the system comprises a non-human animal, and the non-human animal comprises the cells or cells derived from the cell. In certain embodiments, the non-human animal comprises a rodent. In certain embodiments, the rodent comprises a mouse.

In certain embodiments, the system further comprises the inducer.

In certain embodiments, the first expression vector and/or the second expression vector comprise or are derived from a viral vector. In certain embodiments, the viral vector comprises or is derived from a lentiviral vector.

In certain embodiments, the first expression vector comprises a first promoter, and the first promoter is operably linked to a sequence encoding the first regulatory factor. In certain embodiments, the first promoter is a constitutive promoter. In certain embodiments, the first promoter is selected from the group consisting of CMV, EF, SFFV, RSV, PGK and MSCV.

In certain embodiments, the first expression vector comprises or is derived from a pLentis-CMV-IRES-Bsd vector.

In certain embodiments, the second expression vector comprises a second promoter, the second promoter is an inducible promoter that can be activated by the activated first regulatory factor, and wherein the second promoter is operably linked to a sequence encoding the biological macromolecule.

In certain embodiments, the second promoter comprises a TRE promoter.

In certain embodiments, the first expression vector further comprises a first selection marker.

In certain embodiments, the second expression vector further comprises a second selection marker.

In certain embodiments, the first selection marker and/or the second selection marker are each independently selected from one of more of the group consisting of an antibiotic marker and a reporter protein. In certain embodiments, one or more of the report proteins are selected from the group consisting of luciferase, green fluorescent protein, red fluorescent protein, blue fluorescent protein, cyan fluorescent protein and yellow fluorescent protein. In certain embodiments, one or more of the antibiotic markers are selected from the group consisting of Puromycin, Blasticidin, Neomycin, Hygromycin and Bleomycin.

In certain embodiments, the first expression vector further comprises an IRES.

In certain embodiments, the biological macromolecule comprises a cytokine, a chemokine and an antibody. In certain embodiments, one or more of the cytokines are selected from the group consisting of interleukin, interferon, lymphotoxin and colony stimulating factor. In certain embodiments, one or more of the interleukins are selected from the group consisting of IL12, IL2, IL7, IL15 and IL21. In certain embodiments, one or more of the interferons are selected from the group consisting of IPNγ, IFNα and IPNβ. In certain embodiments, the lymphotoxin is selected from the group consisting of LT-α and LT-β. In certain embodiments, one or more of the colony stimulating factors are selected from the group consisting of FMS-associated tyrosine kinase 3 ligand and granulocyte macrophage colony stimulating factor. In certain embodiments, the biological macromolecule comprises a combination selected from the group consisting of 1) IPNγ, IL2 and LT; 2) IL12, GMCSF and IL7; and 3) IL12, FLT3L and IL7.

In certain embodiments, one or more of the inducers are selected from the group consisting of doxycycline and tetracycline.

In another aspect, the present application provides use of the system for preparation of a kit for screening anti-tumor substances.

In certain embodiments, the anti-tumor substance can inhibit the tumor growth and/or cause tumor regression. In certain embodiments, the tumor comprises a malignant tumor. In certain embodiments, the tumor comprises a melanoma.

In certain embodiments, the kit further comprises an instruction. In certain embodiments, the instruction records: 1) providing a cell comprising the first expression vector and the second expression vector in the system; 2) administering the inducer, so that the inducer and the first regulatory factor co-activate the expression of the biological macromolecule encoded by the second expression vector; and 3) detecting the effects of the expression of the biological macromolecule on the growth and/or survival of the tumor, wherein the biological macromolecule is a candidate anti-tumor substance to be screened.

In another aspect, the present application provides a method for regulating the expression and/or activity of the biological macromolecule, comprising: 1) providing a first expression vector encoding a first regulatory factor; 2) providing a second expression vector encoding a biological macromolecule, wherein the expression of the biological macromolecule can be co-regulated by the first regulatory factor expressed by the first expression vector and an inducer; and 3) administering the inducer, so that the inducer and the first regulatory factor co-activate the expression of the biological macromolecule encoded by the second expression vector.

In another aspect, the present application provides a method for screening anti-tumor substances, comprising: 1) providing a cell comprising a first expression vector and a second expression vector, and the first expression encodes a first regulatory factor and the second expression vector encodes a biological macromolecule, wherein an expression of the biological macromolecule can be co-regulated by the first regulatory factor expressed by the first expression vector and an inducer; 2) administering the inducer, so that the inducer and the first regulatory factor co-activate the expression of the biological macromolecule encoded by the second expression vector; and 3) detecting the effects of the expression of the biological macromolecule on the growth and/or survival of the tumor, wherein the biological macromolecule is a candidate anti-tumor substance to be screened.

In certain embodiments, the inducer changes the conformation of the first regulatory factor.

In certain embodiments, the inducer changes the conformation of the first regulatory factor by binding thereto.

In certain embodiments, the inducer binds to the regulatory factor to form a transcription complex, and the transcription complex activates the expression of the biological macromolecule encoded by the second expression vector.

In certain embodiments, the first expression vector and the second expression vector are different expression vectors independent of each other.

In certain embodiments, the cell comprises two or more types of second expression vectors, and the biological macromolecule encoded by each type of second expression vectors is different from the biological macromolecule encoded by at least another type of second expression vectors.

In certain embodiments, the cells comprise the first expression vector and/or the second expression vector.

In certain embodiments, the method further comprises introducing the first expression vector and/or the second expression vector into a cell by means selected from the group consisting of transfection, electroporation and microinjection to obtain a cell comprising the first expression vector and the second expression vector.

In certain embodiments, the cell comprising the first expression vector and the second expression vector comprises two or more types of cells, each type of cells comprises the first expression vector and the second expression vector, and the biological macromolecule encoded by the second expression vector in each type of cells is different from the biological macromolecule encoded by the second expression vector in at least another type of cells.

In certain embodiments, the first expression vector and/or the second expression vector are integrated into the genome of the cell. In certain embodiments, the cell comprises a tumor cell. In certain embodiments, the tumor cell comprises a melanoma cell. In certain embodiments, the melanoma cell comprises a mouse melanoma cell B16.

In certain embodiments, the method further comprises introducing the cell into to a non-human animal.

In certain embodiments, the method comprises mixing and introducing the two or more types of cells into the non-human animal.

In certain embodiments, the introduction method comprises injection. In certain embodiments, the amount of the introduced cells is at least 5^{∗}10⁴ cells per non-human animal. In certain embodiments, the amount of the introduced cells is at least 1^{∗}10⁵ cells per non-human animal.

In certain embodiments, when the cell comprises two or more types of cells, various types of introduced cells are in equal amounts.

In certain embodiments, when the cell comprises two or more types of cells, various types of cells are mixed in equal amount prior to introduction.

In certain embodiments, the non-human animal comprises a rodent. In certain embodiments, the rodent comprises a mouse.

In certain embodiments, the first expression vector and/or the second expression vector comprise or are derived from viral vector. In certain embodiments, the viral vector comprises or is derived from lentiviral vector.

In certain embodiments, the first expression vector comprises a first promoter, and the first promoter is operably linked to a sequence encoding the first regulatory factor. In certain embodiments, the first promoter is a constitutive promoter. In certain embodiments, the first promoter is selected from the group consisting of CMV, EF, SFFV, RSV, PGK and MSCV.

In certain embodiments, the first expression vector comprises or is derived from pLentis-CMV-IRES-Bsd vector.

In certain embodiments, the second expression vector comprises a second promoter, the second promoter is an inducible promoter that can be activated by the activated first regulatory factor, and wherein the second promoter is operably linked to a sequence encoding the biological macromolecule. In certain embodiments, the second promoter comprises a TRE promoter.

In certain embodiments, the first regulatory factor is rtTA, its functional fragment or variant.

In certain embodiments, the first expression vector further comprises a first selection marker. In certain embodiments, the second expression vector further comprises a second selection marker.

In certain embodiments, one or more of the first selection marker and/or the second selection marker are each independently selected from the group consisting of an antibiotic marker and a report protein. In certain embodiments, one or more of the report proteins are selected from the group consisting of luciferase, green fluorescent protein, red fluorescent protein, blue fluorescent protein, cyan fluorescent protein and yellow fluorescent protein. In certain embodiments, one or more of the antibiotic markers are selected from the group consisting of Puromycin, Blasticidin, Neomycin, Hygromycin and Bleomycin.

In certain embodiments, the first expression vector further comprises an IRES.

In certain embodiments, the biological macromolecule encoded by the second expression vector comprises a cytokine, a chemokine and an antibody. In certain embodiments, one or more of the cytokines are selected from the group consisting of interleukin, interferon, lymphotoxin and colony stimulating factor. In certain embodiments, one or more of the interleukins are selected from the group consisting of IL12, IL2, IL7, IL15 and IL21. In certain embodiments, one or more of the interferons are selected from the group consisting of IPNγ, IFNα and IPNβ. In certain embodiments, the lymphotoxin is selected from the group consisting of LT-α and LT-β. In certain embodiments, one or more of the colony stimulating factors are selected from the group consisting of FMS-associated tyrosine kinase 3 ligand and granulocyte macrophage colony stimulating factor. In certain embodiments, the biological macromolecule comprises a combination selected from the group consisting of 1) IPNγ, IL2 and LT; 2) IL12, GMCSF and IL7; and 3) IL12, FLT3L and IL7.

In certain embodiments, the method comprises administering an inducer to the non-human animal. In certain embodiments, one or more of the administering methods are selected from the group consisting of intratumoral, oral, intraperitoneal or subcutaneous administration. In certain embodiments, the inducer is administered at a concentration of 0.5-10g/L.

In certain embodiments, the method comprises administering the inducer to the non-human animal after the cells form a tumor in the non-human animal.

In certain embodiments, one or more of the inducers are selected from the group consisting of doxycycline and tetracycline.

In certain embodiments, the detection comprises: detecting the expression level of the biological macromolecule before administration of the inducer to obtain a control expression level; detecting the expression level of the biological macromolecule after administration of the inducer to obtain an induced expression level; and comparing the control expression level with the induced expression level, wherein the increase of the induced expression level relative to the control expression level indicates that the expression of the biological macromolecule inhibits the growth and/or survival of the tumor.

In certain embodiments, the detection comprises detecting the volume of the tumor before administration of the inducer to obtain a control tumor volume; detecting the volume of the tumor after administration of the inducer to obtain a detected tumor volume; and comparing the control tumor volume with the detected tumor volume, wherein the decrease of the detected tumor volume relative to the control tumor volume indicates that the expression of the biological macromolecule inhibits the growth and/or survival of the tumor.

In another aspect, the present application provides anti-tumor substances screened by the method as provided herein.

In certain embodiments, the anti-tumor substance comprises a cytokine, a chemokine and an antibody. In certain embodiments, one or more of the cytokines are selected from the group consisting of interleukin, interferon, lymphotoxin and colony stimulating factor. In certain embodiments, one or more of the interleukins are selected from the group consisting of IL12, IL2, IL7, IL15 and IL21. In certain embodiments, one or more of the interferons are selected from the group consisting of IPNγ, IFNα and IPNβ. In certain embodiments, the lymphotoxin is selected from the group consisting of LT-α and LT-β. In certain embodiments, one or more of the colony stimulating factors are selected from the group consisting of FMS-associated tyrosine kinase 3 ligand and granulocyte macrophage colony stimulating factor. In certain embodiments, the biological macromolecule comprises a combination selected from the group consisting of 1) IPNγ, IL2 and LT; 2) IL12, GMCSF and IL7; and 3) IL12, FLT3L and IL7.

In another aspect, the present application provides a method for preparing the system, comprising: 1) constructing the first expression vector; and 2) constructing the second expression vector.

In certain embodiments, the method comprises: 3) introducing the first expression vector into one or more types of cells. In certain embodiments, the method comprises: 4) introducing the second expression vector into one or more types of cells. In certain embodiments, the method comprises: 3) introducing the first expression vector and the second expression vector into two or more types of cells, wherein each type of cells comprises the first expression vector and the second expression vector, and the biological macromolecule encoded by the second expression vector in each type of cells is different from the biological macromolecule encoded by the second expression vector in at least another type of cells.

In certain embodiments, each type of cells comprises and only comprises one type of second expression vector.

In certain embodiments, the method further comprises the step of mixing different types of cells. In certain embodiments, when the cell comprises two or more types of cells, the number of each type of cells as introduced is the same.

In certain embodiments, the method comprises the step of 5) introducing the cells into a non-human animal.

In certain embodiments, the non-human animal comprises a rodent. In certain embodiments, the rodent comprises a mouse.

In certain embodiments, wherein the introduction method comprises injection. In certain embodiments, the injection is subcutaneous injection.

In certain embodiments, the method comprises the following step: 6) allowing the non-human animal comprising the cells to express the biological macromolecule encoded by the second expression vector.

In certain embodiments, the expression comprises administration of an inducer. In certain embodiments, one or more of the inducers are selected from the group consisting of doxycycline and tetracycline. In certain embodiments, the inducer is at a concentration of 0.5-10g/L.

In certain embodiments, the inducer is administered after the growth of the tumor.

In certain embodiments, the expression comprises the up-regulation of the expression level of the biological macromolecule. In certain embodiments, the up-regulation is an increase of expression level of the biological macromolecule after administration of the inducer as compared with the expression level of the biological macromolecule before administration of the inducer.

In certain embodiments, the first expression vector and/or the second expression vector are introduced into the cell by means selected from the group consisting of transfection, electroporation and microinjection.

In certain embodiments, the cell comprises a tumor cell. In certain embodiments, the tumor cell comprises a melanoma cell. In certain embodiments, the melanoma cell comprises a mouse melanoma cell B16.

Additional aspects and advantages of the present disclosure will become readily apparent to those skilled in this art from the following detailed description, wherein only illustrative embodiments of the present disclosure are shown and described. Numerous modifications of the embodiments of the disclosure described herein will now occur to those skilled in the art without departing from the disclosure. Accordingly, the drawings and description of the present disclosure are to be regarded as illustrative in nature, but not as restrictive.**Brief Description of the Drawings**

Specific features of the invention as set forth in this application are set forth in the appended claims. The features and advantages of the inventions of the present application can be better understood by referring to the exemplary embodiments and the accompanying drawings. A brief description of the drawing is as follows:
Fig. 1 shows a schematic view of the system and the method for screening the anti-tumor substance as described in the present application.
Fig. 2 shows a schematic structural view of the first expression vector and the second expression vector of the present application.
Fig. 3 shows a schematic view of inducing the GFP expression of the system of the present application in an *ex vivo* cell culture.
Fig. 4 shows a schematic view of inducing the iRFP expression of the system of the present application *in vivo.*
Fig. 5 shows the effect of the virus transfection involved in the system and the method of the present application and the use of doxycycline on the tumorgenesis of the cells.
Fig. 6 shows the therapeutic effect of the system and the method of the present application on the tumors after inducing the expression of IFNα *in vivo.*
Fig. 7 shows the therapeutic effect of the system and the method of the present application on the tumors after inducing the expression of IFNγ *in vivo.*
Fig. 8 shows the therapeutic effect of the system and the method of the present application on the tumors after inducing the expression of IL12 *in vivo.*
Fig. 9 shows the therapeutic effect of the system and the method of the present application on the tumors after inducing the expression of LT *in vivo.*
Fig. 10 shows the therapeutic effect of the system and the method of the present application on the tumors after inducing the expression of IL2 *in vivo.*
Fig. 11 shows the therapeutic effect of the system and the method of the present application on the tumors after inducing the expression of GMCSF *in vivo.*
Fig. 12 shows the therapeutic effect of the system and the method of the present application on the tumors after inducing the expression of IFNγ + IL2 *in vivo.*
Fig. 13 shows the therapeutic effect of the system and the method of the present application on the tumors after inducing the expression of IL12 + IL2 *in vivo.*
Fig. 14 shows the therapeutic effect of the system and the method of the present application on the tumors after inducing the expression of GMCSF + IL2 *in vivo.*
Fig. 15 shows the therapeutic effect of the system and the method of the present application on the tumors after inducing the expression of GMCSF + IL2 *in vivo.*
Fig. 16 shows the therapeutic effect of the system and the method of the present application on the tumors after inducing the expression of IFNγ + IL2 + LT *in vivo.*
Fig. 17 shows the therapeutic effect of the system and the method of the present application on the tumors after inducing the expression of IL12 + GMCSF + IL7 *in vivo.*
Fig. 18 shows the therapeutic effect of the system and the method of the present application on the tumors after inducing the expression of IL12 + FLT3L + IL7 *in vivo.*

### Detailed Description

The embodiments of the present invention are described below by way of specific embodiments, and those skilled in the art can readily appreciate other advantages and effects of the present invention from the disclosure of the present specification. System and Method for Controlled Expression of Biological Macromolecule/Screening of Anti-tumor Substances

The present application provides a system for controlled expression of a biological macromolecule (e.g., a plurality of biological macromolecules or a combination thereof), comprising: 1) a first expression vector encoding a first regulatory factor; and 2) a second expression vector encoding a biological macromolecule, wherein the expression of the biological macromolecule can be co-regulated by the first regulatory factor expressed by the first expression vector and an inducer, wherein the biological macromolecule is a candidate anti-tumor substance to be screened.

The present application further provides a method for regulating the expression and/or the activity of a biological macromolecule, and the method comprises: 1) providing a first expression vector encoding a first regulatory factor; 2) providing a second expression vector encoding a biological macromolecule, wherein the expression of the biological macromolecule can be co-regulated by the first regulatory factor expressed by the first expression vector and an inducer; and 3) administering the inducer, so that the inducer and the first regulatory factor co-activate the expression of the biological macromolecule encoded by the second expression vector.

Meanwhile, the present application provides a system for screening anti-tumor substances, and the system comprises: 1) a first expression vector encoding a first regulatory factor; and 2) a second expression vector encoding a biological macromolecule, wherein the expression of the biological macromolecule can be co-regulated by the first regulatory factor expressed by the first expression vector and an inducer.

The present application further provides a method for screening anti-tumor substances, comprising: 1) providing a cell comprising a first expression vector encoding a first regulatory factor and a second expression vector encoding a biological macromolecule, wherein the expression of the biological macromolecule can be co-regulated by the first regulatory factor expressed by the first expression vector and an inducer; 2) administering the inducer, so that the inducer and the first regulatory factor co-activate the expression of the biological macromolecule encoded by the second expression vector; and 3) detecting the effects of the expression of the biological macromolecule on the growth and/or survival of the tumor, wherein the biological macromolecule is a candidate anti-tumor substance to be screened.

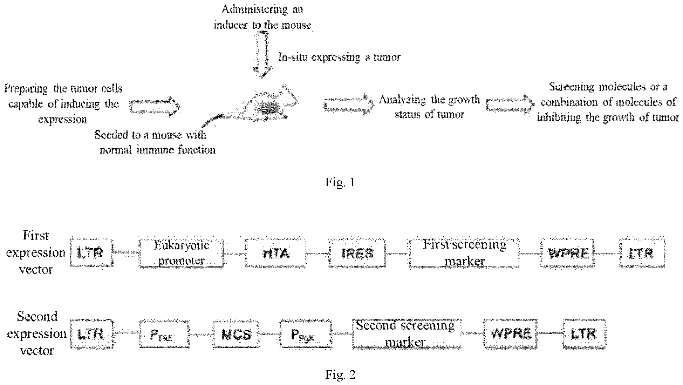

In the present application, for the schematic view of the system and the method for screening the anti-tumor substances, please refer to Fig. 1.

In the present application, the term "first expression vector" refers generally to an expression vector capable of expressing a first regulatory factor of the present application. The first expression vector may express a regulatory factor that can play a regulatory role along with an inducer. For instance, the first expression vector may be a vector pUC57. For instance, the first expression vector may be pLentis-CMV-IRES-Bsd. Also as an example, the first expression vector may be pLentis-CMV-rtTA-IRES-Bsd with a nucleotide sequence as shown in SEQ ID NO.1.

In the present application, the term "first regulatory factor" refers generally to a regulatory factor capable of co-regulating the expression of a biological macromolecule along with an inducer. In the present application, the first regulatory factor may be encoded and/or expressed by the first expression vector. For instance, the first regulatory factor may comprise a transcription factor.

In the present application, the term "transcription factor" refers generally to a protein capable of specifically regulating the transcription level of a specific gene. For instance, the transcription factor may regulate the binding of a ribonucleic acid polymerase (also known as RNA polymerase or RNA synthetase) to a DNA template. The transcription factor may bind to a promoter region upstream of the gene to be regulated (i.e., an upstream transcription factor), and may also be combined with other transcription factor to form a complex of transcription factors to affect the transcription level of the gene to be regulated. Of those, the upstream transcription factor may bind to the upstream of the transcription starting site of the gene to be regulated, thereby facilitating or inhibiting the transcription direction. An inducible transcription factor may activate/inhibit the expression of the gene to be regulated under induction conditions (e.g., in the presence of an inducer).

In certain embodiments, the first regulatory factor comprises an rtTA (reverse tetracycline-controlled transactivator), its functional fragment or variant. For instance, the rtTA protein may be the rtTA of *E. coli.* Also as an example, the rtTA protein may be expressed by the expression vector peTetOn/FKNF (PacI). For instance, the amino acid sequence of rtTA may be as shown in GenBank Accession No. ALK44378.1. In certain embodiments, the first regulatory factor comprises a tTA (tetracycline-controlled transactivator), its functional fragment or variant. For instance, the tTA protein (e.g., the GenBank Accession Number of its amino acid sequence may be ACG80850.1) may be obtained by the fusion of TetR (tetracycline repressor) with an activated domain of a protein VP16. For instance, the TetR may be TetR of *E. Coli.* For instance, the GenBank Accession Number of the amino acid sequence of the TetR may be ADL14074.1, ALS39163.1, ALS39153.1, or AGY30766.1. For instance, the protein VP16 may be derived from a human alpha herpesvirus, and the GenBank Accession Number of its amino acid sequence may be AAA45864.1; and further as an example, the GenBank Accession Number of the amino acid sequence of the protein VP16 may be AAA45863.1. In the present application, the amino acid sequence of rtTA may be as shown in SEQ ID NO.3.

In the present application, the term "functional fragment or variant" may refer to any fragment or variant having the function of the first regulatory factor as described above. For instance, the functional fragment or variant may be a functional fragment or variant of rtTA protein. For instance, the GenBank Accession Number of the amino acid sequence of the functional fragment or variant of the rtTA protein may be ALK44378.1, AIU94979.1, AIU94972.1, AIU94961.1, or ALP32061.1. For instance, the functional fragment or variant may be a functional fragment or variant of the tTA protein. For instance, the GenBank Accession Number of the amino acid sequence of the functional fragment or variant of the tTA protein may be ACG80850.1. For instance, the functional fragment or variant may be a functional fragment or variant of the VP16 protein. The GenBank Accession Number of the amino acid sequence of the functional fragment or variant of the VP16 protein may be AGS36541.1, AFC98774.1, or AEX37895.1. For instance, the functional fragment or variant may have an amino acid sequence that is obtained by substitution, deletion, or addition of one or more amino acid on the basis of the amino acid sequence of the first regulatory factor, while still retains the function of the first regulatory factor. Also as an example, the functional fragment or variant may be an amino acid sequence that has at least about 80% or more (e.g., at least about 80% or more, about 85% or more, about 90% or more, about 95% or more, about 98% or more, about 99% or more) identity to the amino acid sequences of the first regulatory factor, and still retains the function of the first regulatory factor.

In the present application, the term "conformation" refers generally to a spatial conformation of a protein. For instance, a protein is usually a covalent polypeptide chain formed by the end-to-end condensation of amino acids. It may have a specific spatial structure or a three-dimensional structure. This three-dimensional structure may be called the conformation of protein. Changes in protein conformation may cause its activity and/or functional changes.

In the present application, the term "inducer" refers generally to a substance capable of changing the conformation of the protein. In the present application, the inducer may change the spatial conformation of the first regulatory factor. For instance, the inducer may bind to the first regulatory factor, thereby changing the conformation of the first regulatory factor. For instance, the first regulatory factor may be rtTA, its functional fragment or variant, and the inducer may be an antibiotic tetracycline or its derivative (e.g., doxycycline, DOX). At that time, the inducer (e.g., DOX) may bind to rtTA (or its functional fragment or variant) so as to change the conformation of the rtTA (or its functional fragment or variant). In the present application, one or more of the inducers may be selected from the group consisting of doxycycline and tetracycline. For instance, the inducer may be doxycycline (DOX). In the present application, the system can further comprise an inducer. In the present application, the method may further comprise administering (e.g., to a non-human animal) an inducer. For instance, the administration method may be one or more selected from the group consisting of intratumoral, oral, intraperitoneal or subcutaneous administration. In certain embodiments, the inducer may be administered at a concentration of about 0.5-10g/L (e.g., about 0.1-100g/L, about 0.1-90g/L, about 0.5-80g/L, about 0.5-70g/L, about 0.5-60g/L, about 0.5-50g/L, about 0.5-40g/L, about 0.5-30g/L, about 0.5-20g/L, about 0.5-10g/L, about 0.5-5g/L, about 0.5-2.5g/L or about 0.5-2g/L).

In the present application, the term "tetracycline" refers generally to a class of broad-spectrum antibiotics with perhydro-phenanthrene core. These antibiotics are widely used for infections caused by Gram-positive and Gram-negative bacteria, intracellular mycoplasma, chlamydia, and Rickettsia. The chemical formula of the tetracycline may be C₂₂H₂₄N₂O₈. For instance, tetracycline may be extracted from Strepto-myces aureofaciens; or, tetracycline may be produced by Actinomycetes bacteria of the genus Streptomyces.

In the present application, the term "doxycycline" refers generally to one of tetracyclines, also known as Doxitard, Doxy, Liviatin, Dox, Medomycin, Monodoxin, etc. The doxycycline may be used to treat infections caused by chlamydia and mycoplasma. It is pale yellow or yellow crystalline powder, smelly, bitter; and soluble in water or methanol, slightly soluble in ethanol or acetone, and insoluble in chloroform. The doxycycline hydrochloride salt is also a commonly used antibiotic.

In the present application, the term "transcription complex" refers generally to a complex composed of one or more of promoter, RNA polymerase and various other transcription factors. The transcription complex may regulate the transcription level of the gene to be regulated. For instance, the transcription complex may regulate the transcription level of a downstream gene by binding to a promoter and/or an enhancer. In the present application, the inducer may bind to the regulatory factor to form a transcription complex (e.g., DOX may bind to rtTA to form a transcription complex comprising rtTA with changed conformation), and the transcription complex may activate the expression of the biological macromolecule encoded by the second expression vector.

In the present application, the term "the second expression vector" refers generally to an expression vector encoding the biological macromolecule of the present application. The second expression vector may encode and/or express the biological macromolecule. For instance, the second expression vector may be pLentis-PTRE-MCS-PGK-PURO with a nucleotide sequence as shown in SEQ ID NO.2. In the present application, the expression of the biological macromolecule may be co-regulated by the first regulatory factor expressed by the first expression vector and the inducer. For example, the biological macromolecule encoded by the second expression vector may be co-regulated by the first regulatory factor expressed by the first expression vector and the inducer. Of those, the inducer may bind to the first regulatory factor to change the conformation of the first regulatory factor, so that the inducer binds to the first regulatory factor to form a transcription complex; and the transcription complex in turn may activate the expression of the biological macromolecule encoded by the second expression vector.

In the present application, the "co-regulate/co-regulating" may mean that the expression of the second regulatory factor may be regulated if and only if the first regulatory factor and the inducer are correctly present. For instance, when the first regulatory factor and the inducer exist at the same time, the inducer may change the conformation of the first regulatory factor by binding to the first regulatory factor, and the first regulatory factor with changed conformation forms a transcription complex along with the inducer. The transcription complex may affect the expression of the second regulatory factor. In the present application, when only the first regulatory factor is present, or when only the inducer is present, or when the inducer does not result in a conformational change of the first regulatory factor, or when the inducer may not bind to the first regulatory factor to form a transcription complex capable of regulating the expression of the second regulatory factor, the expression of the second regulatory factor may not be co-regulated by the first regulatory factor and the inducer.

In the present application, the term "co-active/co-activating" generally means that the expression of downstream genes is activated when two different substances exist at the same time. For instance, the two substances may bind to each other to form a transcription complex, which initiate the transcription of the downstream gene. For instance, the inducer and the regulatory factor may bind to each other to form a transcription complex, which in turn activates the expression of the biological macromolecule encoded by the second expression vector.

In certain embodiments, the inducer may be DOX, and the first expression vector may be pLentis-CMV-IRES-Bsd vector. DOX may bind to the first regulatory factor, rtTA, expressed by the first expression vector, pLentis-CMV-IRES-Bsd vector, and after binding, the conformation of rtTA is changed to form a transcription complex comprising rtTA with changed conformation, which may activate the expression of the biological macromolecule encoded by the second expression vector (e.g., cytokine). Two or more types of different second expression vectors may encode two or more biological macromolecules that are different from each other. In certain embodiments, each of the second expression vectors may encode a biological macromolecule, which is different from the biological macromolecule encoded by at least another (or even any other) type of second expression vector.

In the present application, the first expression vector and/or the second expression vector may comprise or be derived from viral vectors. In the present application, the term "viral vector" refers generally to a vector tool that infects cells with a virus so as to introduce the target genetic material into the cells. For instance, the viral vector may be selected from the group consisting of retroviral vector, lentiviral vector and adenoviral vector. For instance, the viral vector may comprise or be derived from lentiviral vector. For instance, the first expression vector comprises or is derived from pLentis-CMV-IRES-Bsd vector. In the present application, the structures of the first expression vector and the second expression vector may be as shown in Fig. 2.

In the present application, the term "biological macromolecule" refers generally to a protein molecule or a fused or conjugated protein comprising of protein molecules, etc. In the present application, the biological macromolecule may be encoded and/or expressed by the second expression vector. For instance, the expression and/or level of the biological macromolecule may be co-regulated by the first regulatory factor expressed by the first expression vector and an inducer. In the present application, the biological macromolecule may be cytokine, chemokine or antibody. In certain embodiments, the biological macromolecule further comprises a protein secreted out of the cells. For instance, the biological macromolecule may comprise salivary amylase, pepsin, digestive enzymes, insulin and glucagon.

In the present application, the term "cytokine" refers generally to small polypeptides secreted mainly by immune cells and capable of regulating the function of cells. The cytokine plays an important regulatory role in the interaction between the cells, the growth and differentiation of cells. For instance, one or more of the cytokines may be selected from the group consisting of interleukin, interferon, lymphotoxin and colony stimulating factor. For instance, one or more of the interleukins may be selected from the group consisting of IL12, IL2, IL7, IL15 and IL21. For instance, one or more of the interferons may be selected from the group consisting of IFNγ, IFNα and IPNβ. For instance, the lymphotoxin is selected from the group consisting of LT-α and LT-β. For instance, one or more of the colony stimulating factors may be selected from the group consisting of FMS-associated tyrosine kinase 3 ligand and granulocyte macrophage colony stimulating factor.

In the present application, the term "chemokine" refers generally to some low molecular weight protein capable of attracting white blood cells to move to the infected site, and may also be called as chemotactic hormone, chemerin, or chemohormone. Common structural characteristics among chemokine proteins comprise low molecular weight (about 8-10 KDalton) and the presence of four position-conserved cysteine residues to ensure its tertiary structure. For instance, the chemokine may comprise CCL family, CXCL family, XCL family and CX3C family. The chemokine may lead to a migration of chemotactic cells. Some chemokines control the chemotaxis of immune cells during immune surveillance, such as inducing lymphatic cells to lymph nodes.

In the present application, the term "antibody" refers generally to immunoglobulin (briefly, Ig) which is an immunoglobulin molecule composed of two pairs of identical polypeptide chains (each pair has a "light" (L) chain and a "heavy" (H) chain). Antibody light chains may be classified as κ and λ light chains. The heavy chains may be classified as µ, δ, γ, α or ε, and the antibody isotypes are defined as IgM, IgD, IgG, IgA and IgE. Within the light and heavy chains, variable regions and constant regions are linked via "J" region(s) with about 12 or more amino acids, and the heavy chains further comprise "D" regions with about 3 or more amino acids. The heavy chain consists of heavy chain variable regions (VHs) and heavy chain constant regions (CHs). The heavy chain constant region consists of three domains (CH1, CH2 and CH3). Each light chain consists of light chain variable regions (VLs) and light chain constant regions (CLs). The light chain constant region consists of one domain CL. The constant regions of antibodies may mediate the binding of immunoglobulins to host tissues or factors, comprising various cells of the immune system (e.g., effector cells) to the first component (C1q) of the classical complement system. The VH and VL regions may be further subdivided into regions with high variability (called complementarity determining regions (CDRs)) having more conserved regions called framework regions (FRs) interspersed therebetween. Various VHs and VLs consist of 3 CDRs and 4 FRs arranged in the order of FR1, CDR1, FR2, CDR2, FR3, CDR3, FR4 from amino terminus to carboxy terminus, respectively. The variable regions (VHs and VLs) of various heavy/light chain pairs form antibody binding sites, respectively. In the present application, the first expression vector and the second expression vector are different expression vectors independent of each other. For example, the system can comprise 1 (or about 1, 2, 3, 4, 5, or more) type of first expression vector and 1 (or about 1, 2, 3, 4, 5 or more) type of second expression vector.

In the present application, the system may comprise two or more (e.g., about 2, 3, 4, 5, 6 or more) types of second expression vectors, and the biological macromolecule encoded by each type of second expression vector is different from the biological macromolecule encoded by at least another (e.g., about 1, 2, 3, 4, 5, 6, or more) type of second expression vector in the system. For instance, the system may comprise two or more (e.g., about 2, 3, 4, 5, 6 or more) types of second expression vectors, and the cytokines encoded by each type of second expression vectors (e.g., the first cytokine and the second cytokine expressed by each type of second expression vectors, respectively) are different from the cytokines (e.g., the third cytokine) encoded by at least another (e.g., about 1, 2, 3, 4, 5, 6 or more) types of second expression vectors in the system.

For example, the system may comprise two or more types of second expression vectors, wherein one of the two or more types of second expression vectors encodes the first cytokine, and the other of the two or more types of second expression vectors encodes the second cytokine which is different from the first cytokine. Also as an example, the system may comprise two or more types of second expression vectors, wherein each type of second expression vectors encode the first cytokine, the second cytokine, the third cytokine ...... and so on, respectively. The first cytokine, the second cytokine, the third cytokine and the like are different from each other.

In the present application, the system may comprise a cell comprising the first expression vector and/or the second expression vector. For instance, the system may comprise two or more types of cells (e.g., about 2, 3, 4, 5, 6 or more), each type of cells comprises the first expression vector and the second expression vector, and the biological macromolecule encoded by the second expression vector in each type of cells (e.g., the first cytokine expressed by each type of second expression vector) is different from the biological macromolecule (e.g., the second cytokine) encoded by at least another types of second expression vector in the system.

In the present application, the term "cell" refers generally to cell comprising the first expression vector and one type of second expression vector. For instance, the cell may comprise the first expression vector, and comprises and only comprises the second expression vector encoding one biological macromolecule (e.g., the first cytokine).

For example, the system may comprise two or more types of cells comprising the second expression vector, wherein the second expression vector in one of two or more types of cells encodes the first cytokine, and the second expression vector of the other of two or more types of cells encode the second cytokine which is different from the first cytokine. Also as an example, the system may comprise many types of cells comprising different second expression vectors, wherein each type of second expression vectors encodes the first cytokine, the second cytokine, the third cytokine ...... and so on, respectively. The first cytokine, the second cytokine, the third cytokine and the like are different from each other.

For example, the system may comprise two or more (e.g., about 2, 3, 4, 5, 6 or more) types of cells. Since the biological macromolecule encoded by the second expression vector in each type of cells (e.g., the first cytokine) is different from the biological macromolecule encoded by the second expression vector of at least another type of cell in the system (e.g., the second cytokine which is different from the first cytokine), the system may simultaneously express two or more (e.g., about 2, 3, 4, 5, 6 or more) types of biological macromolecules through the inducer and the regulatory factor (e.g., simultaneously expressing the first cytokine and the second cytokine). Also as an example, the system may simultaneously express three types of biological macromolecules through the inducer and the regulatory factor, e.g., simultaneously express the first cytokine, the second cytokine, and the third cytokine, wherein the first cytokine, the second cytokine and the third cytokine are different from each other.

In the present application, the first cytokine, the second cytokine and the third cytokine may be independently selected from the group consisting of 1) IPNγ, IL2 and LT; 2) IL12, GMCSF and IL7; and 3) IL12, FLT3L and IL7.

In the present application, the first cytokine and the second cytokine may be independently selected from the group consisting of 1) IFNγ and IL2; 2) IL12 and IL2; 3) GMCSF and IL2; and 4) IL12 and GMCSF.

For example, the system may comprise three types of cells, wherein the second expression vector in one type of cell encodes IPNγ, the second expression vector in one type of cell encodes IL2, and the second expression vector in the other type of cell encodes LT. For another example, the system may be more than four types of cells, wherein the second expression vector in two or more types of cells encodes IPNγ, the second expression vector in one type of cell encodes IL2, and the second expression vector in the other type of cell encodes LT. For another example, the system may comprise four or more types of cells, wherein the second expression vectors in two or more types of cells encode IPNγ, the second expression vectors in two or more types of cells encode IL2, and the second expression vectors in two or more types of cells encode LT.

In the present application, the first expression vector and/or the second expression vector may be integrated into the genome of the cell.

In the present application, the system may be *ex vivo* system or *in vitro* system.

In the present application, the method may be an *ex vivo* method, or an *in vitro* method.

In the present application, the cells may comprise tumor cells. For instance, the tumor cells may be the same type of tumor cells. In the present application, the tumor cells may be the same type of tumor cells, and each of the tumor cells comprises the first and the second expression vectors encoding different biological macromolecules, respectively. For example, the tumor cells (e.g., the first tumor cell, such as, mouse melanoma cell B16) may comprise the first expression vector and a type of second expression vector encoding the biological macromolecule (e.g., the first cytokine); the tumor cells (e.g., the first tumor cells, such as, mouse melanoma cell B16) may further comprise the first expression vector and another type of second expression vector encoding the biological macromolecule (e.g., the second cytokine which is different from the first cytokine). In the present application, the tumor cells may comprise melanoma cell. For instance, the melanoma cell may comprise a mouse melanoma cell B16.

In the present application, the first expression vector and/or the second expression vector may be introduced into the cells to obtain a cell comprising the first expression vector and the second expression vector by means selected from the group consisting of transfection, electroporation and microinjection.

In the present application, the system may comprise a non-human animal, and the non-human animal comprises the cells or cells derived therefrom. For instance, the non-human animal may comprise a rodent. For instance, the rodent may comprise a mouse.

In the present application, the method may comprise introducing the cells into the non-human animal. For instance, the introduction method may comprise injection. Meanwhile, in the present application, the two or more types of cells may be mixed and introduced into the non-human animal. For instance, the amount of the introduced cells is at least about 5^{∗}10⁴ cells per non-human animal (e.g., at least about 1^{∗}10⁴, 2^{∗}10⁴, 3^{∗}10⁴, 4^{∗}10⁴ 5^{∗}10⁴, 6^{∗}10⁴, 7^{∗}10⁴, 8^{∗}10⁴, 9^{∗}10⁴, 1^{∗}10⁵, 2^{∗}10⁵ or more). For instance, when the cell comprises two or more (e.g., about 2, 3, 4, 5, 6 or more) types of cells, the amount of each introduced cell may be the same. For instance, when the cell comprises two or more (e.g., about 2, 3, 4, 5, 6 or more) types of cells, each type of cells may be mixed and introduced in equal quantity (alternatively, e.g., optionally and/or in different quantity).

In the present application, the method also may comprise administering an inducer to the non-human animal after the cells form a tumor in the non-human animal.

In the present application, the term "promoter" refers generally to a DNA sequence which is located upstream of the transcription start site and has RNA polymerase-recognizing, binding and transcription-initiating functions. The promoter may comprise a conserved sequence that specifically binds to an RNA polymerase and is required by transcription initiation.

In the present application, the term "first promoter" generally refers to a promoter comprised in the first expression vector and operably linked to a sequence encoding the first regulatory factor. For example, the first promoter may be a constitutive promoter. In the present application, the term "constitutive promoter" generally refers to a promoter that may initiate expression in all tissues. The regulation of constitutive promoters is not affected by external conditions, and the expression of the promoted genes is continuous. For example, the first promoter may be selected from: CMV, EF, SFFV, RSV, PGK, and MSCV.

In the present application, the term "second promoter" generally refers to a promoter which is comprised in the second expression vector, may be activated by the activated first regulatory factor, and operably linked to a sequence encoding the biological macromolecule. For example, the second promoter may be an inducible promoter. In the present application, the term "inducible promoter" generally refers to a promoter that may drastically and rapidly stimulate the transcription activity when stimulated under induction conditions, and may rapidly regulate the expression of the induced gene. For example, the second promoter may comprise a TRE promoter.

In the present application, the first expression vector may further comprise IRES. The IRES is an internal ribosome entry site, and may allow the ribosomes leaving the mRNA after translation to bind to the mRNA to be translated again.

In the present application, the term "selection marker" refers generally to a specific marker for screening if the expression vector has been transferred to the target cells. For instance, the selection marker may comprise antibiotic marker and report proteins. In the present application, the first expression vector may further comprise the first selection marker. And/or, the second expression vector may further comprise a second selection marker. For instance, the first selection marker may indicate if the first expression vector has been transferred into the cells. Also as an example, the second selection marker may indicate if the second expression vector has been transferred into the cells. In the present application, the first selection marker may be different from the second selection marker. For instance, one or more of the first selection marker and/or the second selection marker may each independently be selected from the group consisting of antibiotic marker and report proteins.

In the present application, the term "antibiotic marker" refers generally to an antibiotic as the selection marker. For instance, one or more of the antibiotic markers may be selected from the group consisting of Puromycin, Blasticidin, Neomycin, Hygromycin and Bleomycin.

In the present application, the term "report protein" refers generally to a protein as selection marker, e.g., fluorescent proteins. For instance, one or more of the report proteins may be one or more selected from the group consisting of luciferase, green fluorescent protein, red fluorescent protein, blue fluorescent protein, cyan fluorescent protein and yellow fluorescent protein.

In the method of the present application, the detection may comprise detecting the expression level of the biological macromolecule before administration of the inducer to obtain an control expression level; detecting the expression level of the biological macromolecule after administration of the inducer to obtain an induced expression level; comparing the control expression level with the induced expression level, wherein the increase of the induced expression relative to the control expression level (e.g., an increase of 1 time, 2 times, 3 times, 4 times, 5 times, 6 times, 7 times, 8 times, 9 times or 10 times or more) indicates that the expression of the biological macromolecule inhibits the growth and/or survival of the tumor.

In the method of the present application, the detection may comprise detecting the volume of the tumor before administration of the inducer to obtain a control tumor volume; detecting the volume of the tumor after administration of the inducer to obtain a detected tumor volume; and comparing the control tumor volume with the detected tumor volume, wherein the decrease of the detected tumor volume relative to the control tumor volume (e.g., a decrease of about 5%, 10%, 20%, 30%, 40%, 50%, 60%, 70%, 80%, 90%, 95%, 99% or more) indicates that the expression of the biological macromolecule inhibits the growth and/or survival of the tumor.

### Anti-Tumor Substances and Kit for Screening Anti-Tumor Substances

The present application provides use of the system in preparation of a kit for screening anti-tumor substances. The present application also provides a kit comprising the system for screening anti-tumor substances.

The present application further provides anti-tumor substances screened by the method. In the present application, the tumor may comprise a malignant tumor, e.g., a melanoma.

In the present application, the term "anti-tumor substance" refers generally to substances capable of inhibiting the tumor growth and/or causing tumor regression. For instance, the anti-tumor substances may comprise cytokine, chemokine and antibody. In certain embodiments, one or more of the cytokines may be selected from the group consisting of interleukin, interferon, lymphotoxin and colony stimulating factor. For instance, one or more of the interleukins may be selected from the group consisting of IL12, IL2, IL7, IL15 and IL21. For instance, one or more of the interferons may be selected from the group consisting of IPNγ, IFNα and IPNβ. For instance, the lymphotoxin is selected from the group consisting of LT-α and LT-β. For instance, one or more of the colony stimulating factors may be selected from the group consisting of FMS-associated tyrosine kinase 3 ligand and granulocyte macrophage colony stimulating factor. In certain embodiments, the anti-tumor substance comprises a combination selected from the group consisting of 1) IPNγ, IL2 and LT; 2) IL12, GMCSF and IL7; and 3) IL12, FLT3L and IL7.

In the present application, the kit may comprise the system or a portion thereof.

In the present application, the kit may comprise the first expression vector and the second expression vector. For instance, the kit may comprise a cell comprising the first expression vector and the second expression vector. In the present application, the kit may further comprise an inducer.

In the present application, the kit may further comprise a detection reagent for detecting the expression level of the biological macromolecule encoded by the second expression vector. For instance, the detection reagent may comprise a reagent capable of detecting the transcription level and/or the protein expression level of the biological macromolecule. For instance, the detection reagent may comprise a reagent capable of detecting the activity of the biological macromolecule. For instance, the detection reagent may comprise a reagent capable of detecting the transcription level and/or the protein expression level of one or more of IFNγ, IL2, LT, GMCSF, IL7 and FLT3L. Also as an example, the detection reagent may comprise a reagent capable of detecting the activity of one or more of IFNγ, IL2, LT, GMCSF, IL7 and FLT3L.

For example, the detection reagent may comprise one or more of a primer for the amplification of the biological macromolecule, a DNA polymerase and a PCR buffer. For instance, the detection reagent may comprise a material or a reagent required by qPCR reaction, e.g., Green I, DNA polymerase, dNTP, amplification buffer; or may comprise a sequence-specific oligonucleotide probe which is optically active when hybridized with amplification products, such as, fluorescent probes. For another example, the detection reagent may comprise a nucleic acid polymerase, a reverse transcriptase, dNTP, Mg²⁺ ion, a buffer required for the amplification reaction, and/or a reporter for detecting the amplification product.

In the present application, the detection reagent may further comprise a marker capable of marking the biological macromolecule. For instance, the marker may comprise TaqMan probe, TaqMan Tamara probe, TaqMan MGB probe or Lion probe. Also as an example, the marker may be an optically active reporting agent, which may comprise SYBR Green, SYBR Blue, DAPI, propidium iodide, ethidium bromide, acridine, proflavine, acridine orange, acridine yellow and/or fluorescent coumarin.

In the present application, the kit may further comprise an instruction. For instance, the instruction may record: 1) providing the first expression vector and the second expression vector of the system; 2) administering the inducer, so that the inducer and the first regulatory factor co-activate the expression of the biological macromolecule encoded by the second expression vector; and 3) detecting the effects of the expression of the biological macromolecule on the growth and/or survival of the tumor, wherein the biological macromolecule is a candidate anti-tumor substance to be screened.

For example, the first expression vector and/or the second expression vector may be comprised in a cell. The kit may comprise such cells. The cells may be Eukaryotic cells.

### Method for Preparing the System for Controlled Expression of Biological Macromolecule/Screening of Anti-Tumor Substances

In another aspect, the present application further provides a method for preparing the system of the present application, comprising: 1) constructing the first expression vector; and 2) constructing the second expression vector.

In the present application, the preparation method may further comprise: 3) introducing the first expression vector into one or more types of cells; and/or 3) introducing the first expression vector and the second expression vector into two or more types of cells, wherein each type of cells comprises the first expression vector and the second expression vector, and the biological macromolecule encoded by the second expression vector in each type of cells is different from the biological macromolecule encoded by the second expression vector in at least another type of cells; and/or 4) introducing the second expression vector into one or more types of cells.

In the preparation method of the present application, each type of cells may comprise and only comprise one type of second expression vectors.

The preparation method of the present application may further comprise the following steps: mixing various types of cells. For instance, when the cell comprises two or more types of cells, the amounts of each type of cells as introduced may be the same.

In the present application, the preparation method may further include: 5) introducing the cells into a non-human animal. Of those, the non-human animal may comprise a rodent. For instance, the rodent may comprise a mouse. Of those, the introduction method may comprise injection. For instance, the injection may be subcutaneous injection.

In the present application, the preparation method may further include: 6) allowing the non-human animal comprising the cells to express the biological macromolecule encoded by the second expression vector. Of those, the expression may be carried out by administering an inducer. Of those, one or more of the inducers may be selected from the group consisting of doxycycline and tetracycline. For instance, the inducer may be administered at a concentration of 0.5-10g/L (e.g., may be 0.1-100g/L, 0.1-90g/L, 0.5-80g/L, 0.5-70g/L, 0.5-60g/L, 0.5-50g/L, 0.5-40g/L, 0.5-30g/L, 0.5-20g/L, 0.5-10g/L, 0.5-5g/L, 0.5-2.5g/L or 0.5-2g/L). For instance, the concentration may be 2g/L. And/or, the inducer may be administered after the growth of the tumor.

In the preparation method of the present application, the expression may comprise the up-regulation of the expression level of the biological macromolecule. For instance, the up-regulation may be an increase (e.g., an increase of about 1 time, 2 times, 3 times, 4 times, 5 times, 6 times, 7 times, 8 times, 9 times or 10 times or more) of the expression level of the biological macromolecule after administration of the inducer as compared with the expression level of the biological macromolecule before administration of the inducer.

In the preparation method of the present application, the first expression vector and/or the second expression vector may be introduced into the cells by means selected from the group consisting of transfection, electroporation and microinjection. Of those, the cells may comprise tumor cells. For instance, the tumor cells may comprise melanoma cell, e.g., the melanoma cell may comprise a mouse melanoma cell B16.

In the present application, the term "about" refers generally to a variation within 0.5%-10% of a given value, such as, a variation within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of a given value.

The present application relates to the following embodiments:
1 A system for a controlled expression of a biological macromolecule, comprising:
   1) a first expression vector encoding a first regulatory factor; and
   2) a second expression vector encoding a biological macromolecule, wherein an expression of the biological macromolecule can be co-regulated by the first regulatory factor expressed by the first expression vector and an inducer, and the biological macromolecule is a candidate anti-tumor substance to be screened.
2 A system for screening anti-tumor substances, comprising:
   1) a first expression vector encoding a first regulatory factor; and
   2) a second expression vector encoding a biological macromolecule, wherein an expression of the biological macromolecule can be co-regulated by the first regulatory factor expressed by the first expression vector and an inducer.
3 The system according to any one of embodiments 1-2, wherein the first regulatory factor comprises a transcription factor.
4 The system according to any one of embodiments 1-3, wherein the inducer changes the conformation of the first regulatory factor.
5 The system according to embodiment 4, wherein the inducer changes the conformation of the first regulatory factor by binding to the first regulatory factor.
6 The system according to any one of embodiments 4-5, wherein the inducer binds to the regulatory factor to form a transcription complex, and the transcription complex activates the expression of the biological macromolecule encoded by the second expression vector.
7 The system according to any one of embodiments 1-6, wherein the first expression vector and the second expression vector are different expression vectors independent of each other.
8 The system according to any one of embodiments 1-7, wherein the system comprises two or more types of second expression vectors, and the biological macromolecule encoded by each type of second expression vectors is different from the biological macromolecule encoded by at least another type of second expression vectors in the system.
9 The system according to any one of embodiments 1-8, wherein the system comprises a cell, and the cell comprises the first expression vector and/or the second expression vector.
10 The system according to embodiment 9, wherein the system comprises two or more types of cells, each type of cells comprises the first expression vector and the second expression vector, and the biological macromolecule encoded by the second expression vector in each type of cells is different from the biological macromolecule encoded by the second expression vector in at least another type of cells.
11 The system according to any one of embodiments 9-10, wherein the first expression vector and/or the second expression vector are integrated into the genome of the cell.
12 The system according to any one of embodiments 9-11, wherein the cell comprises a tumor cell.
13 The system according to embodiment 12, wherein the tumor cell comprises a melanoma cell.
14 The system according to embodiment 13, wherein the melanoma cell comprises a mouse melanoma cell B16.
15 The system according to any one of embodiments 9-14, wherein the system comprises a non-human animal, and the non-human animal comprises the cells or cells derived from the cells.
16 The system according to embodiment 15, wherein the non-human animal comprises a rodent.
17 The system according to embodiment 16, wherein the rodent comprises a mouse.
18 The system according to any one of embodiments 1-17, wherein the system further comprises the inducer.
19 The system according to any one of embodiments 1-18, wherein the first expression vector and/or the second expression vector comprise or are derived from viral vector.
20 The system according to embodiment 19, wherein the viral vector comprises or is derived from lentiviral vector.
21 The system according to any one of embodiments 1-20, wherein the first expression vector comprises a first promoter operably linked to a sequence encoding the first regulatory factor.
22 The system according to embodiment 21, wherein the first promoter is a constitutive promoter.
23 The system according to embodiment 22, wherein the first promoter is selected from the group consisting of CMV, EF, SFFV, RSV, PGK and MSCV.
24 The system according to any one of embodiments 21-23, wherein the first expression vector comprises or is derived from pLentis-CMV-IRES-Bsd vector.
25 The system according to any one of embodiments 1-24, wherein the second expression vector comprises a second promoter, the second promoter is an inducible promoter that can be activated by the activated first regulatory factor, and wherein the second promoter is operably linked to a sequence encoding the biological macromolecule.
26 The system according to embodiment 25, wherein the second promoter comprises a TRE promoter.
27 The system according to any one of embodiments 1-26, wherein the first regulatory factor is rtTA, its functional fragment or variant.
28 The system according to any one of embodiments 1-27, wherein the first expression vector further comprises a first selection marker.
29 The system according to any one of embodiments 1-28, wherein the second expression vector further comprises a second selection marker.
30 The system according to any one of embodiments 28-29, wherein one or more of the first selection marker and/or the second selection marker are each independently selected from the group consisting of antibiotic marker and report proteins.
31 The system according to embodiment 30, wherein one or more of the report proteins are selected from the group consisting of luciferase, green fluorescent protein, red fluorescent protein, blue fluorescent protein, cyan fluorescent protein and yellow fluorescent protein.
32 The system according to embodiment 30, wherein one or more of the antibiotic markers are selected from the group consisting of Puromycin, Blasticidin, Neomycin, Hygromycin and Bleomycin.
33 The system according to any one of embodiments 1-32, wherein the first expression vector further comprises an IRES.
34 The system according to any one of embodiments 1-33, wherein the biological macromolecule comprises a cytokine, chemokine and antibody.
35 The system according to embodiment 34, wherein one or more of the cytokines are selected from the group consisting of interleukin, interferon, lymphotoxin and colony stimulating factor.
36 The system according to embodiment 35, wherein one or more of the interleukins are selected from the group consisting of IL12, IL2, IL7, IL15 and IL21.
37 The system according to embodiment 35, wherein one or more of the interferons are selected from the group consisting of IPNγ, IFNα and IPNβ.
38 The system according to embodiment 35, wherein the lymphotoxin is selected from the group consisting of LT-α and LT-β.
39 The system according to embodiment 35, wherein one or more of the colony stimulating factors are selected from the group consisting of FMS-associated tyrosine kinase 3 ligand and granulocyte macrophage colony stimulating factor.
40 The system according to any one of embodiments 1-39, wherein one or more of the inducers are selected from the group consisting of doxycycline and tetracycline.
41 Use of the system according to any one of embodiments 1-40 in preparation of a kit for screening anti-tumor substances.
42 The use according to embodiment 41, wherein the anti-tumor substance can inhibit the tumor growth and/or cause tumor regression.
43 The use according to any one of embodiments 41-42, wherein the tumor comprises a malignant tumor.
44 The use according to any one of embodiments 41-43, wherein the tumor comprises a melanoma.
45 The use according to any one of embodiments 41-44, wherein the kit further comprises an instruction.
46 The use according to embodiment 45, wherein the instruction records:
   1) providing a cell comprising a system which comprises the first expression vector and the second expression vector;
   2) administering the inducer, so that the inducer and the first regulatory factor co-activate the expression of the biological macromolecule encoded by the second expression vector; and
   3) detecting the effects of the expression of the biological macromolecule on the growth and/or survival of the tumor, wherein the biological macromolecule is a candidate anti-tumor substance to be screened.
47 A method for regulating the expression and/or activity of a biological macromolecule, comprising:
   1) providing a first expression vector encoding a first regulatory factor;
   2) providing a second expression vector encoding a biological macromolecule, wherein the expression of the biological macromolecule can be co-regulated by the first regulatory factor expressed by the first expression vector and an inducer; and
   3) administering the inducer, so that the inducer and the first regulatory factor co-activate the expression of the biological macromolecule encoded by the second expression vector.
48 A method for screening anti-tumor substances, comprising:
   1) providing a cell comprising a first expression vector and a second expression vector, and the first expression encodes a first regulatory factor and the second expression vector encoding a biological macromolecule, wherein an expression of the biological macromolecule can be co-regulated by the first regulatory factor expressed by the first expression vector and an inducer;
   2) administering the inducer, so that the inducer and the first regulatory factor co-activate the expression of the biological macromolecule encoded by the second expression vector; and
   3) detecting the effects of the expression of the biological macromolecule on the growth and/or survival of the tumor.
49 The method according to any one of embodiment 47 or 48, wherein the inducer changes the conformation of the first regulatory factor.
50 The method according to any one of embodiments 47-49, wherein the inducer changes the conformation of the first regulatory factor by binding thereto.
51 The method according to any one of embodiments 47-50, wherein the inducer binds to the regulatory factor to form a transcription complex, and the transcription complex activates the expression of the biological macromolecule encoded by the second expression vector.
52 The method according to any one of embodiments 47-51, wherein the first expression vector and the second expression vector are different expression vectors independent of each other.
53 The method according to any one of embodiments 48-52, wherein the cell comprises two or more types of second expression vectors, and the biological macromolecule encoded by each type of second expression vectors is different from the biological macromolecule encoded by at least another type of second expression vectors.
54 The method according to any one of embodiments 48-53, wherein the cell comprises the first expression vector and/or the second expression vector.
55 The method according to any one of embodiments 48-54, further comprising introducing the first expression vector and/or the second expression vector into the cells to obtain a cell comprising the first expression vector and the second expression vector by means selected from the group consisting of transfection, electroporation and microinjection.
56 The method according to any one of embodiments 48-55, wherein the cell comprising the first expression vector and the second expression vector comprises two or more types of cells, each type of cells comprises the first expression vector and the second expression vector, and the biological macromolecule encoded by the second expression vector in each type of cells is different from the biological macromolecule encoded by the second expression vector in at least another type of cells.
57 The method according to any one of embodiments 48-56, wherein the first expression vector and/or the second expression vector are integrated into the genome of the cell.
58 The method according to any one of embodiments 48-57, wherein the cell comprises a tumor cell.
59 The method according to embodiment 58, wherein the tumor cell comprises a melanoma cell.
60 The method according to embodiment 59, wherein the melanoma cell comprises a mouse melanoma cell B16.
61 The method according to any one of embodiments 48-60, further comprising introducing the cell into to a non-human animal.
62 The method according to any one of embodiments 56-61, comprising mixing and introducing the two or more types of cells into the non-human animal.
63 The method according to embodiment 62, wherein the introduction method comprises injection.
64 The method according to any one of embodiments 62-63, wherein the amount of the introduced cells is at least 5^{∗}10⁴ cells per non-human animal.
65 The method according to embodiment 64, wherein the amount of the introduced cells is at least 1^{∗}10⁵ cells per non-human animal.
66 The method according to any one of embodiments 56-65, wherein when the cell comprises two or more types of cells, various types of introduced cells are in equal amounts.
67 The method according to any one of embodiments 56-66, wherein when the cell comprises two or more types of cells, various types of cells are mixed in equal amount prior to introduction.
68 The method according to any one of embodiments 61-67, wherein the non-human animal comprises a rodent.
69 The method according to embodiment 68, wherein the rodent comprises a mouse.
70 The method according to any one of embodiments 47-69, wherein the first expression vector and/or the second expression vector comprise or are derived from viral vector.
71 The method according to embodiment 70, wherein the viral vector comprises or is derived from lentiviral vector.
72 The method according to any one of embodiments 47-71, wherein the first expression vector comprises a first promoter operably linked to a sequence encoding the first regulatory factor.
73 The method according to embodiment 72, wherein the first promoter is a constitutive promoter.
74 The method according to embodiment 73, wherein the first promoter is selected from the group consisting of CMV, EF, SFFV, RSV, PGK and MSCV.
75 The method according to any one of embodiments 47-74, wherein the first expression vector comprises or is derived from pLentis-CMV-IRES-Bsd vector.
76 The method according to any one of embodiments 47-75, wherein the second expression vector comprises a second promoter, the second promoter is an inducible promoter that can be activated by the activated first regulatory factor, and wherein the second promoter is operably linked to a sequence encoding the biological macromolecule.
77 The method according to embodiment 75, wherein the second promoter comprises a TRE promoter.
78 The method according to any one of embodiments 47-77, wherein the first regulatory factor is rtTA, its functional fragment or variant.
79 The method according to any one of embodiments 47-78, wherein the first expression vector further comprises a first selection marker.
80 The method according to any one of embodiments 47-80, wherein the second expression vector further comprises a second selection marker.
81 The method according to any one of embodiments 78-79, wherein one or more of the first selection marker and/or the second selection marker are each independently selected from the group consisting of antibiotic marker and report proteins.
82 The method according to embodiment 81, wherein one or more of the report proteins are selected from the group consisting of luciferase, green fluorescent protein, red fluorescent protein, blue fluorescent protein, cyan fluorescent protein and yellow fluorescent protein.
83 The method according to embodiment 81, wherein one or more of the antibiotic markers are selected from the group consisting of Puromycin, Blasticidin, Neomycin, Hygromycin and Bleomycin.
84 The method according to any one of embodiments 47-83, wherein the first expression vector further comprises an IRES.
85 The method according to any one of embodiments 47-84, wherein the biological macromolecule encoded by the second expression vector comprises a cytokine, chemokine and antibody.
86 The method according to embodiment 85, wherein one or more of the cytokines are selected from the group consisting of interleukin, interferon, lymphotoxin and colony stimulating factor.
87 The method according to embodiment 85, wherein one or more of the interleukins are selected from the group consisting of IL12, IL2, IL7, IL15 and IL21.
88 The method according to embodiment 85, wherein one or more of the interferons are selected from the group consisting of IPNγ, IFNα and IPNβ.
89 The method according to embodiment 85, wherein the lymphotoxin is selected from the group consisting of LT-α and LT-β.
90 The method according to embodiment 85, wherein one or more of the colony stimulating factors are selected from the group consisting of FMS-associated tyrosine kinase 3 ligand and granulocyte macrophage colony stimulating factor.
91 The method according to any one of embodiments 61-90, comprising administering an inducer to the non-human animal.
92 The method according to embodiment 91, wherein one or more of the administering methods are one or more selected from the group consisting of intratumoral, oral, intraperitoneal or subcutaneous administration.
93 The method according to any one of embodiments 91-92, wherein the inducer is administered at a concentration of 0.5-10g/L.
94 The method according to any one of embodiments 61-94, wherein the inducer is administered to the non-human animal after the cells form a tumor in the non-human animal.
95 The method according to any one of embodiments 91-94, wherein one or more of the inducers are selected from the group consisting of doxycycline and tetracycline.
96 The method according to any one of embodiments 48-95, wherein the detection comprises detecting the expression level of the biological macromolecule before administration of the inducer to obtain a control expression level; detecting the expression level of the biological macromolecule after administration of the inducer to obtain an induced expression level; comparing the control expression level with the induced expression level, wherein the increase of the induced expression relative to the control expression level indicates that the expression of the biological macromolecule inhibits the growth and/or survival of the tumor.
97 The method according to any one of embodiments 48-96, wherein the detection comprises detecting the volume of the tumor before administration of the inducer to obtain a control tumor volume; detecting the volume of the tumor after administration of the inducer to obtain a detected tumor volume; comparing the control tumor volume with the detected tumor volume, wherein a decrease of the detected tumor volume relative to the control tumor volume indicates that the expression of the biological macromolecule inhibits the growth and/or survival of the tumor.
98 An anti-tumor substance screened by the method according to any one of embodiments 48-97.
99 The anti-tumor substances according to embodiment 98, comprising cytokine, chemokine and antibody.
100 The anti-tumor substance according to embodiment 99, wherein one or more of the cytokines are selected from the group consisting of interleukin, interferon, lymphotoxin and colony stimulating factor.
101 The anti-tumor substance according to embodiment 100, wherein one or more of the interleukins are selected from the group consisting of IL12, IL2, IL7, IL15 and IL21.
102 The anti-tumor substance according to embodiment 100, wherein one or more of the interferons are selected from the group consisting of IPNγ, IFNα and IPNβ.
103 The anti-tumor substance according to embodiment 100, wherein the lymphotoxin is selected from the group consisting of LT-α and LT-β.
104 The anti-tumor substance according to embodiment 100, wherein one or more of the colony stimulating factors are selected from the group consisting of FMS-associated tyrosine kinase 3 ligand and granulocyte macrophage colony stimulating factor.
105 The anti-tumor substance according to any one of embodiments 98-104, which are one or more selected from the group consisting of:
   1) IPNγ, IL2 and LT;
   2) IL12, GMCSF and IL7; and
   3) IL12, FLT3L and IL7.
106 A method for preparing the system according to embodiments 1-40, comprising:
   1) constructing the first expression vector; and
   2) constructing the second expression vector.
107 The method according to embodiment 106, comprising:
   3) introducing the first expression vector into one or more types of cells.
108 The method according to any one of embodiments 106-107, comprising:
   4) introducing the second expression vector into one or more types of cells.
109 The method according to any one of embodiments 106-108, comprising:
   3) introducing the first expression vector and the second expression vector into two or more types of cells, wherein each type of cells comprises the first expression vector and the second expression vector, and the biological macromolecule encoded by the second expression vector in each type of cells is different from the biological macromolecule encoded by the second expression vector in at least another type of cells.
110 The method according to any one of embodiments 106-109, wherein each type of cells comprises and only comprises one type of second expression vectors.
111 The method according to any one of embodiments 106-110, further comprising the step of mixing various types of cells.
112 The method according to any one of embodiments 106-111, wherein when the cell comprises two or more types of cells, the amount of each type of cells as introduced is the same.
113 The method according to any one of embodiments 106-112, comprising the following step:
   5) introducing the cells into a non-human animal.
114 The method according to embodiment 113, wherein the non-human animal comprises a rodent.
115 The method according to embodiment 114, wherein the rodent comprises a mouse.
116 The method according to any one of embodiments 113-115, wherein the introduction method comprises injection.
117 The method according to embodiment 116, wherein the injection is subcutaneous injection.
118 The method according to any one of embodiments 113-117, comprising the following step:
   6) allowing the non-human animal containing the cells to express the biological macromolecule encoded by the second expression vector.
119 The method according to embodiment 118, wherein the expression is carried out by administration of an inducer.
120 The method according to embodiment 119, wherein one or more of the inducers are selected from the group consisting of doxycycline and tetracycline.
121 The method according to embodiment 120, wherein the inducer is at a concentration of 0.5-10g/L.
122 The method according to any one of embodiments 119-121, wherein the inducer is administered after the growth of the tumor.
123 The method according to any one of embodiments 109-122, the expression comprises the up-regulation of the expression level of the biological macromolecule.
124 The method according to embodiment 123, wherein the up-regulation is an increase of the expression level of the biological macromolecule after administration of the inducer as compared with the expression level of the biological macromolecule before administration of the inducer.
125 The method according to any one of embodiments 109-124, wherein the first expression vector and/or the second expression vector are introduced into the cells by means selected from the group consisting of transfection, electroporation and microinjection.
126 The method according to any one of embodiments 107-125, wherein the cell comprises a tumor cell.
127 The method according to embodiment 126, wherein the tumor cell comprises a melanoma cell.
128 The method according to embodiment 127, wherein the melanoma cell comprises a mouse melanoma cell B16.

Without intending to be bound by any theory, the following examples are merely intended to illustrate the working modes of the device, the method and the system of the present application, rather than to limit the scope of the invention of the present application. **Examples**

Reagents: DMEM media, 1640 media and fetal bovine serum are purchased from Lifetechnologies Company; cell culture bottles and culture plates are purchased from Corning Company; doxycycline (DOX) is purchased from Sangong Biotech (Shanghai) Co., Ltd.; Puromycin and Blasticidin are purchased from Chemicon Company; restriction endonuclease is purchased from Takara and NEB Company; ligase is purchased from NEB Company; DNA polymerase is purchased from Takara Company; plasmid extraction kit and gel recovery kit are purchased from OmegaBiotech Company; the synthesis of primers is performed by Sangong Biotech (Shanghai) Co., Ltd.; and the synthesis and sequencing of genes are performed by Lifetechnologies Company.

### Example 1: Construction of the first expression vector

The DNA coding sequence of rtTA (the amino acid sequence is shown in SEQ ID NO.3) was synthesized, which had BamHI and EcoRI sites at both ends, and the synthetic product was ligated within vector pUC57. This vector was digested with the following digestion system: 6µg of plasmid, 4 µl of digestion buffer, 1 µl of BamHI, 1 µl of EcoRI. The mixture was supplemented with water to a total volume of 40 µl, and stood at 37°C for 12 hours. 4.4 µl of 10x loading buffer was added into the EP tube, and an electrophoresis was performed on a 1% agarose gel. After electrophoresis, the rtTA fragments were recovered until usage.

The vector pLentis-CMV-IRES-Bsd was digested in the EP tube with the following digestion system: 2 µg of plasmid, 3 µl of digestion buffer, 1 µl of BamHI, 1 µl of EcoRI. The mixture was supplemented with water to a total volume of 30 µl, and stood at 37°C for 12 hours. 3.3 µl of 10x loading buffer was added into the EP tube, and an electrophoresis was performed on a 1% agarose gel. After electrophoresis, the vector fragments were recovered until usage.

pLentis-CMV-IRES-Bsd was ligated to rtTA with a system as follows: 2 µl of pLentis-CMV-IRES-Bsd, 2 µl of rtTA, 1 µl of ligase buffer, 0.5 µl of T4DNA ligase, 4.5 µl of water. The mixture was subject to linking at room temperature for 4 hr. Then, the linking system was subject to transformation of *E. coli* competent cells. At the next day, colonies were picked from the transformed plates, and placed in LB media at 37°C in a shaker overnight. Plasmids were extracted from the cultured bacteria with a plasmid extraction kit, and digested for identifying whether the fragments were successfully ligated into the vector. The vector was sent for sequencing to determine that the first expression vector pLentis-CMV-rtTA-IRES-Bsd was successfully constructed (the nucleotide sequence of the first expression vector was as shown in SEQ ID NO.1).

### Example 2: Preparation of cells comprising the first expression vector

### 2.1 A virus comprising the first expression vector was prepared by the following method:

1. The cultured 293FT cells were digested, counted, and then plated to a 10cm Petri dish at 3 x 10⁶ cells/well with the media volume of 10ml. 2. At the next night, the cells were observed for their status, and transfected if they were in good conditions. Chloroquine was added to the culture plate to a final concentration of 25 µM. Sterilized water and plasmids (pMD2.G5µg + pSPAX215µg + pLentis-CMV-rtTA-IRES-Bsd20µg) were added into a tube to a total volume of 1045 µl, and then 155 µl of 2MCaCl₂ was added. The mixture was uniformly mixed, and finally 1200 µl of 2x HBS was dropwise added with shaking. After the completion of addition, the mixture was quickly added into the cell culture wells, and gently shaken to uniform. 3. On the third morning, the cells were observed for their status, and the media was replaced with 10ml of fresh DMEM media. 4. On the fifth morning, the cells were observed for their status, and the supernatant in the Petri dish was collected, filtered with a 0.45µm filter, and then centrifuged at 50000g in a highspeed centrifuge tube for 2 hr. The supernatant was carefully discarded, and the liquid was carefully removed with absorbent paper. Then, the pellet was resuspended with 500 µl of HBSS, dissolved for 2 hr, and divided into small tubes for storage at -70°C.

### 2.2 The first expression vector virus was used to transfect tumor cells by the following method:

The cultured mouse melanoma cells B16 were digested and seeded at 10⁵ cells/well into a 6-well plate, and the culture volume was 1 ml. After 24 hours, 10 µl of the first expression vector virus was added, and cultured in an incubator for additional 24 hours. The supernatant was discarded and replaced with a fresh media to continue culture. After the cells grow over the bottom, they were transferred to a culture flask, and blasticidin was added at a sutiable concentration to continue culture. The media was replaced every two days, and blasticidin was maintained at a concentration of 8 µg/ml. After one week of screening, the surviving cells were those that stably expressed the regulatory protein. These cells are named B16 (rtTA).

### Example 3

### 3.1 Construction of the second expression vector encoding green fluorescent protein (GFP)

A PCR reaction was performed by using a primer and a template with GFP gene to amplify the GFP gene. The PCR conditions followed the instruction of PrimeStarHSDNApolymerase. The PCR was recovered with a gel recovery kit after agarose gel electrophoresis, and then digested with BamHI and EcoRI with the following digestion system: 30µg of PCR recovery product, 4 µl of digestion buffer, 1 µl of BamHI, 1 µl of EcoRI. The mixture was supplemented with water to a total volume of 40 µl, and stood at 37°C for 12 hr. 4.4 µl 10x loading buffer was added into the EP tube, and an electrophoresis was performed with 1% agarose gel, and then the GFP gene fragments were recovered until usage. The regulating expression vector pLentis-PTRE-MCS-PGK-PURO (the nucleotide sequence of the vector was as shown in SEQ ID NO.2) was digested with the following system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube. An electrophoresis was performed with 1% agarose gel, and after the electrophoresis the vector fragments were recovered until usage. The pLentis-PTRE-MCS-PGK-PURO were ligated with GFP with the following ligation system: 2 µl of pLentis-PTRE-MCS-PGK-PURO, 2 µl of GFP, 1 µl of ligase buffer, 0.5 µl of T4DNA ligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vectors were sequenced to determine that the second expression vector pLentis-PTRE-GFP-PGK-PURO was successfully constructed.

### 3.2 Preparation of cells which regularly express the GFP

The GFP expression vector virus was prepared by the same method as that of the first expression vector virus. The cultured B16 (rtTA) tumor cells were digested, and seeded at 10⁵ cells/well into a 6-well plate, and the culture volume was 1 ml. After 24 hr, 10 µl of the second regulating expression vector virus was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were those that regularly expressed the GFP. These cells are named B16 (rtTA) -GFP.

### 3.3 Demonstration of effect of regulating expression

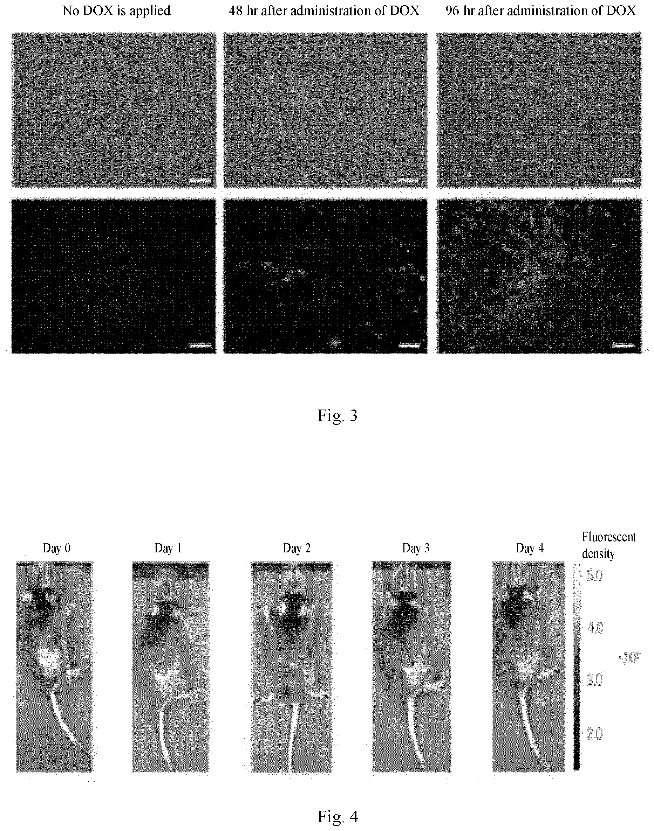

B16 (rtTA) -GFP were plated at 2^{∗}10⁴ cells/well into a 24-well plate, while 100ng/ml of doxycycline (DOX) was added to induce expression and not added to the control well. At different time points, the cells were observed with a fluorescent microscope for their fluorescent expression to determine the effect of induction (Fig. 3). Of those, the top row of Fig. 3 shows the observation result of brightness; and the bottom row shows the observation result of Fluorescence.

### Example 4

### 1. Construction of the second expression vector encoding the red fluorescent protein (iRFP)

iRFP gene was synthesized with BamHI and EcoRI restriction sites at both ends, and then digested by BamHI and EcoRI with the following digestion system: 5µg of iRFP plasmid, 4 µl of digestion buffer, 1 µl of BamHI, 1 µl of EcoRI. The mixture was supplemented with water to a total volume of 40 µl, and stood at 37°C for 12 hr. 4.4 µl 10x loading buffer was added into the EP tube and an electrophoresis was performed with 1% agarose gel, and after the electrophoresis, the iRFP gene fragments were recovered until usage. The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube and an electrophoresis was performed with 1% agarose gel, and after the electrophoresis, the vector fragments were recovered until usage. The pLentis-PTRE-MCS-PGK-PURO were ligated with iRFP with the following ligation system: 2 µl of pLentis-PTRE-MCS-PGK-PURO, 2 µl of iRFP, 1 µl of ligase buffer, 0.5 µl of T4DNA ligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-iRFP-PGK-PURO.

### 2. Preparation of cells for regulating the expression of iRFP

An iRFP expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of iRFP. These cells are named B16 (rtTA) -iRFP.

### 3. Verification of the effect of regulating expression

B16 (rtTA) -iRFP cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks. After the tumor grew, the mice were fed with water containing 2 g/L of doxycycline (DOX). The mouse was observed by and in vovo imaging equipment at different time points for the fluorescence expression of iRFP, thereby determining the working effect of this system *in vivo* (Fig. 4). The results indicate that the administration of doxycycline to the mouse can induce the expression of the target gene (e.g., iRFP) at the tumor site at a relatively high expression level, and maintain the high expression level of the target gene at the tumor site for a period of time.

### Example 5

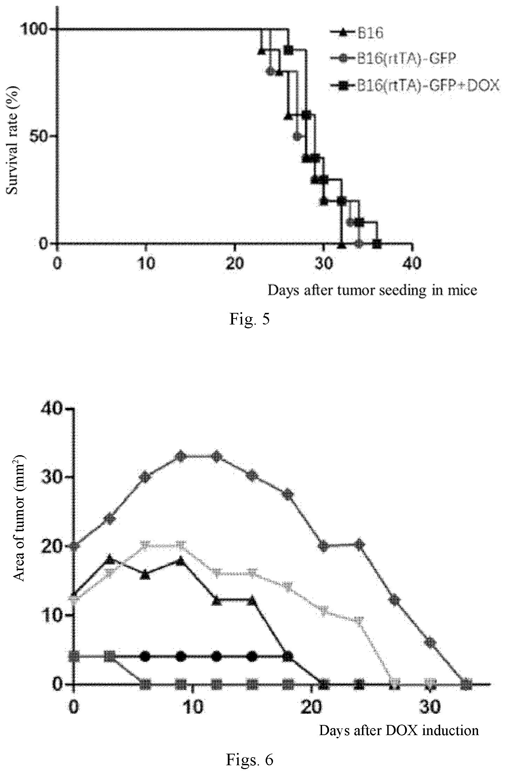

B16 (rtTA) -GFP cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (20 mice in total). Ten of the mice were fed with common water, and the other ten were fed with water containing 2 g/L of doxycycline after the tumor grew. The mouse seeded with untransfected B16 cells was used as the control to record the survival rate of the mouse. The results show that the use of viral transfection and doxycycline did not affect the tumorigenicity of B16 cells (Fig. 5, wherein each broken line represents the tumor size in a mouse).

### Example 6

### 1. Construction of the second expression vector encoding interferon α (IFNα)

An IFNα (GenBank: NM_010502) gene coding region sequence was synthesized with BamHI and EcoRI restriction sites at both ends, and then digested by BamHI and EcoRI with the following digestion system: 5µg of IFNα plasmid, 4 µl of digestion buffer, 1 µl of BamHI, 1 µl of EcoRI. The mixture was supplemented with water to a total volume of 40 µl, and stood at 37°C for 12 hr. 4.4 µl 10x loading buffer was added into the EP tube, and an electrophoresis was performed with 1% agarose gel, and after the electrophoresis, the IFNα gene fragment was recovered until usage. The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube, and an electrophoresis was performed with 1% agarose gel, and after the electrophoresis, the vector fragments were recovered for use. pLentis-PTRE-MCS-PGK-PURO and IFNα were ligated with the following ligation system: 2 µl of pLentis-PTRE-MCS-PGK-PURO, 2 µl of IFNα, 1 µl of ligase buffer, 0.5 µl of T4DNA ligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-IFNα-PGK-PURO.

### 2. Preparation of cells for regulating the expression of IFNα

An IFNα expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of IFNα. These cells are named B16 (rtTA) -IFNα.

### 3. Evaluation of tumor therapeutic effect of IFNα

B16 (rtTA) -IFNα cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (10 mice in total). The mice were fed with water containing 2 g/L of doxycycline. The mouse tumor growth and tumor clearance were recorded, and number of mice with tumor cleared/total number of mice is equal to 8/10. The results show that IFNα can induce the tumor regression in some mice (Fig. 6, wherein each broken line represents the *in vivo* tumor area in a mouse). The results of Fig. 6 also verify that the system of the present invention is feasible.

### Example 7

### 1. Construction of the second expression vector of encoding interferon γ (IPNγ)

An IFNγ (GenBank: NM_008337) gene encoding region sequence was synthesized with BamHI and EcoRI restriction sites at both ends, and then digested by BamHI and EcoRI with the following digestion system: 5µg of IFNγ plasmid, 4 µl of digestion buffer, 1 µl of BamHI, 1 µl of EcoRI. The mixture was supplemented with water to a total volume of 40 µl, and stood at 37°C for 12 hr. 4.4 µl 10x loading buffer was added into the EP tube and an electrophoresis was performed with 1% agarose gel, and after the electrophoresis, the IFNγ gene fragments were recovered until usage. The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube and an electrophoresis was performed with 1% agarose gel, and after the electrophoresis, the vector fragments were recovered until usage. pLentis-PTRE-MCS-PGK-PURO and IFNγ were ligated with the following ligation system: 2 µl of pLentis-PTRE-MCS-PGK-PURO, 2 µl of IPNγ, 1 µl of ligase buffer, 0.5 µl of T4DNAligase,4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-IFNγ-PGK-PURO.

### 2. Preparation of cells for regulating the expression of IFNγ

An IFNγ expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of IFNγ. These cells are named B16 (rtTA) -IFNγ.

### 3. Evaluation of Tumor Therapeutic Effect of IFNγ

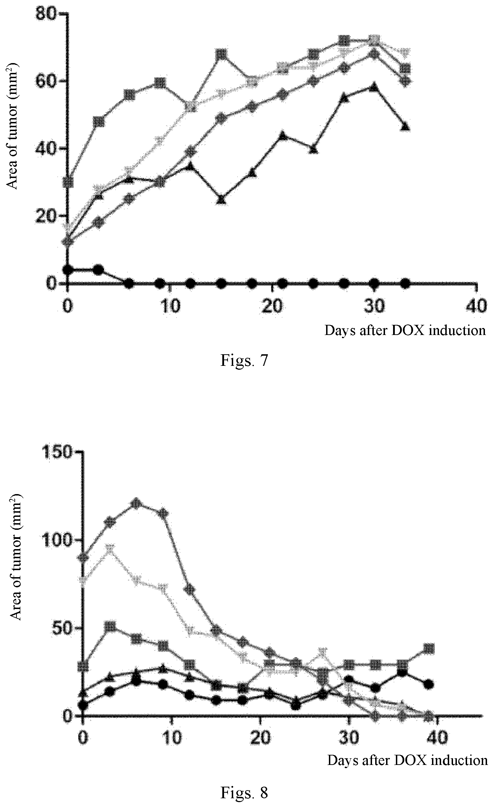

B16 (rtTA)-IFNγ cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (10 mice in total). The mice were fed with water containing 2 g/L of doxycycline after the tumor grew. The mouse tumor growth and tumor clearance were recorded, and number of mice with tumor cleared/total number of mice is equal to 3/10. The results show that the IFNγ can induce the regression of the tumor in some mice (Fig. 7, wherein each broken line represents the *in vivo* tumor area in a mouse). The results of Fig. 7 also verified that the system of the present invention is feasible.

### Example 8

### 1. Construction of the second expression vector of encoding Interleukin 12 (IL12)

An IL12 gene encoding region sequence was synthesized, which included two subunits IL12α (GenBank: NM_008351) and IL12β (GenBank: NM_001303244) linked by a T2A sequence therebetween. The synthesized gene had BamHI and EcoRI restriction sites at the both ends, and then were digested by BamHI and EcoRI with the following digestion system: 5µg of IL12 plasmid, 4 µl of digestion buffer, 1 µl of BamHI, 1 µl of EcoRI. The mixture was supplemented with water to a total volume of 40 µl, and stood at 37°C for 12 hr. 4.4 µl 10x loading buffer was added into the EP tube and an electrophoresis was performed with 1% agarose gel, and after the electrophoresis, the IL12 gene fragments were recovered until usage. The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube and an electrophoresis was performed with 1% agarose gel, and after the electrophoresis, the vector fragments were recovered until usage.

pLentis-PTRE-MCS-PGK-PURO and IL12 were ligated with the following ligation system: 2 µl of pLentis-PTRE-MCS-PGK-PURO,2 µl of IL12, 1 µl of ligase buffer, 0.5 µl of T4DNAligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-IL12-PGK-PURO.

### 2. Preparation of cells for regulating the expression of IL12

An IL12 expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of IL12. These cells are named B16 (rtTA) -IL12.

### 3. Evaluation of Tumor Therapeutic Effect of IL12

B16 (rtTA)-IL12 cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (10 mice in total). The mice were fed with water containing 2 g/L of doxycycline after the tumor grew. The mouse tumor growth and tumor clearance were recorded, and number of mice with tumor cleared/total number of mice is equal to 5/10. The results show that the IL12 can induce the tumor regression in some mice (Fig. 8, wherein each broken line represents the *in vivo* tumor area in a mouse).

### Example 9

### 1. Construction of the second expression vector of encoding lymphotoxinlymphotoxin (LT)

An LT gene encoding region was synthesized which included two subunits LTα (GenBank: NM_010735) and ETβ (GenBank: NM_008518) linked by a P2A sequence. The synthesized gene had BamHI and EcoRI restriction sites at both ends, and then were digested by BamHI and EcoRI with the following digestion system: 5µg of LT plasmid, 4 µl of digestion buffer, 1 µl of BamHI, 1 µl of EcoRI. The mixture was supplemented with water to a total volume of 40 µl, and stood at 37°C for 12 hr. 4.4 µl of 10x loading buffer was added into the EP tube and an electrophoresis was performed with 1% agarose gel, and after the electrophoresis, the LT gene fragments were recovered until usage.

The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube and an electrophoresis was performed with 1% agarose gel, and after the electrophoresis, the vector fragments were recovered until usage. pLentis-PTRE-MCS-PGK-PURO and LT were ligated with the following system: 2 µl of pLentis-PTRE-MCS-PGK-PURO, 2 µl of LT, 1 µl of ligase buffer, 0.5 µl of T4DNA ligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-LT-PGK-PURO.

### 2. Preparation of cells for regulating the expression of LT

An LT expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of LT. These cells are named B16 (rtTA) -LT.

### 3. Evaluation of Tumor Therapeutic Effect of LT

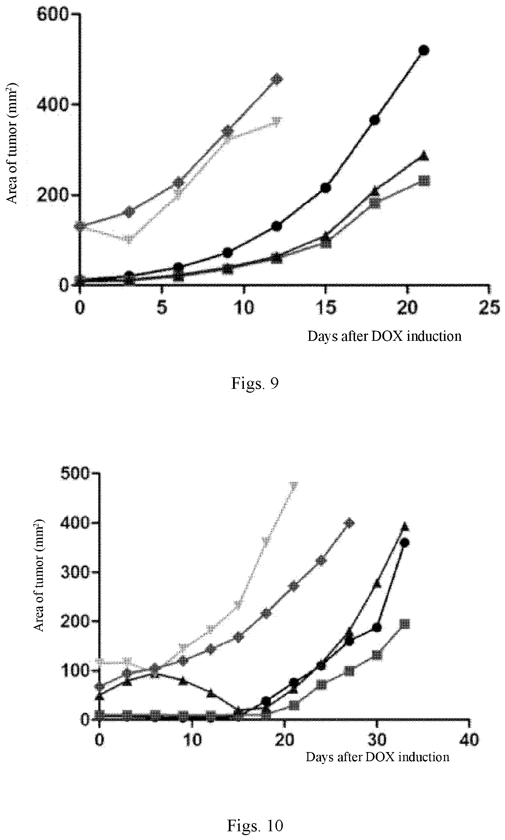

B16 (rtTA)-LT cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (10 mice in total). The mice were fed with water containing 2 g/L of doxycycline. The mouse tumor growth and tumor clearance were recorded, and number of mice with tumor cleared/total number of mice is equal to 0/10. The results show that the LT cannot induce the tumor regression in mouse (Fig. 9, wherein each broken line represents the *in vivo* tumor area in a mouse).

### Example 10

### 1. Construction of the second expression vector of encoding Interleukin 2 (IL2)

An IL2 (GenBank: NM_008366) gene encoding region was synthesized. The synthesized gene had BamHI and EcoRI restriction sites at both ends, and then were digested by BamHI and EcoRI with the following digestion system: 5µg of IL2 plasmid, 4 µl of digestion buffer, 1 µl of BamHI, 1 µl of EcoRI. The mixture was supplemented with water to a total volume of 40 µl, and stood at 37°C for 12 hr. 4.4 µl of 10x loading buffer was added into the EP tube and an electrophoresis was performed with 1% agarose gel, and after the electrophoresis, the IL2 gene fragments were recovered until usage. The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl of 10x loading buffer was added into the EP tube and an electrophoresis was performed with 1% agarose gel, and after the electrophoresis, the vector fragments were recovered until usage. pLentis-PTRE-MCS-PGK-PURO and IL2 were ligated with the following ligation system: 2 µl of pLentis-PTRE-MCS-PGK-PURO, 2 µl of IL2, 1 µl of ligase buffer, 0.5 µl of T4DNA ligase,4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-IL2-PGK-PURO.

### 2. Preparation of cells for regulating the expression of IL2

An IL2 expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of IL2. These cells are named B16 (rtTA) -IL2.

### 3. Evaluation of Tumor Therapeutic Effect of IL2

B16 (rtTA)-IL2 cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (10 mice in total). The mice were fed with water containing 2 g/L of doxycycline after the tumor grew. The mouse tumor growth and tumor clearance were recorded, and number of mice with tumor cleared/total number of mice is equal to 0/10. The results show that the IL2 cannot induce the tumor regression in some mice (Fig. 10, wherein each broken line represents the in vivo tumor area in a mouse).

### Example 11

### 1. Construction of the second expression vector of encoding granulocyte macrophage colony stimulating factor (GMCSF)

A GMCSF (GenBank: NM_009969) gene encoding region was synthesized and the synthesized gene had BamHI and EcoRI restriction sites at both ends, and then were digested by BamHI and EcoRI with the following digestion system: 5µg of GMCSF plasmid, 4 µl of digestion buffer, 1 µl of BamHI, 1 µl of EcoRI. The mixture was supplemented with water to a total volume of 40 µl, and stood at 37°C for 12 hr. 4.4 µl of 10x loading buffer was added into the EP tube and an electrophoresis was performed with 1% agarose gel, and after the electrophoresis, the GMCSF gene fragments were recovered until usage.

The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl of 10x loading buffer was added into the EP tube and an electrophoresis was performed with 1% agarose gel, and after the electrophoresis, the vector fragments were recovered until usage. pLentis-PTRE-MCS-PGK-PURO and GMCSF were ligated with the following ligation system: 2 µl of pLentis-PTRE-MCS-PGK-PURO, 2 µl of GMCSF, 1 µl of ligase buffer, 0.5 µl of T4DNA ligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-GMCSF-PGK-PURO.

### 2. Preparation of cells for regulating the expression of GMCSF

A GMCSF expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were cells capable of regulating the expression of GMCSF. These cells are named B16 (rtTA) -GMCSF.

### 3. Evaluation of Tumor Therapeutic Effect of GMCSF

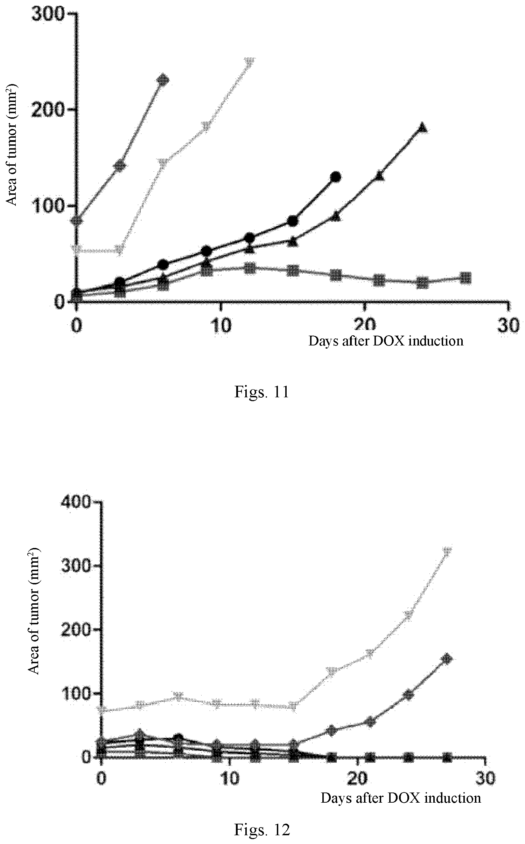

B16 (rtTA)-GMCSF cells in the logarithmic growth phase were digested, diluted with HBSS to 2 x 10⁶ cells/ml, and injected at a dose of 50 µl with a 1 ml injector to the right dorsal side of a C57BL/6 female mouse at 8-10 weeks (10 mice in total). The mice were fed with water containing 2 g/L of doxycycline. The mouse tumor growth and tumor clearance were recorded, and number of mice with tumor cleared/total number of mice is equal to 0/10. The results show that the GMCSF cannot induce the tumor regression in some mice (Fig. 11, wherein each broken line represents the in vivo tumor area in a mouse).

### Example 12. Evaluation of the tumor therapeutic effect of a combination of IFNγ + IL2

B16 (rtTA)-IFNγ and B16 (rtTA)-IL2 cells in the logarithmic growth phase were digested, and resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (12 mice in total). The mice were fed with water containing 2 g/L of doxycycline after the tumor grew. The mouse tumor growth and tumor clearance were recorded, and number of mice with tumor cleared/total number of mice is equal to 5/12. The results show that the combination of IFNγ + IL2 can induce the tumor regression in some mice (Fig. 12, wherein each broken line represents the in vivo tumor area in a mouse).

### Example 13. Evaluation of the tumor therapeutic effect of a combination of IL12 + IL2

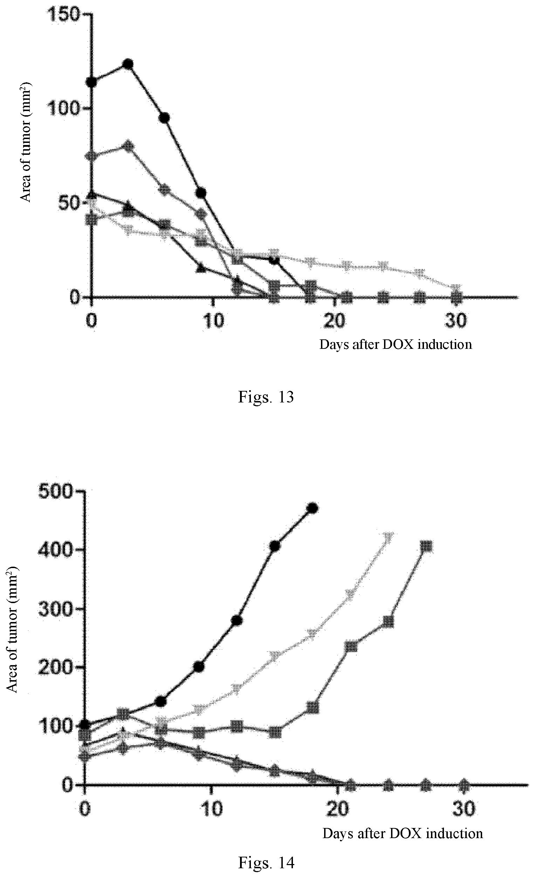

B16 (rtTA)-IL12 and B16 (rtTA)-IL2 cells in the logarithmic growth phase were digested, and resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (15 mice in total). The mice were fed with water containing 2 g/L of doxycycline after the tumor grew. The mouse tumor growth and tumor clearance were recorded, and number of mice with tumor cleared/total number of mice is equal to 15/15. The results show that the combination of IL12 + IL2 can induce the tumor regression in all the tested mice (Fig. 13, wherein each broken line represents the in vivo tumor area in a mouse).

### Example 14. Evaluation of the tumor therapeutic effect of a combination of GMCSF + IL2

B16 (rtTA)-GMCSF and B16 (rtTA)-IL2 cells in the logarithmic growth phase were digested, and resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (15 mice in total). The mice were fed with water containing 2 g/L of doxycycline after the tumor grew. The mouse tumor growth and tumor clearance were recorded, and number of mice with tumor cleared/total number of mice is equal to 6/15. The results show that the combination of GMCSF + IL2 can induce the tumor regression in some tested mice (Fig. 14, wherein each broken line represents the in vivo tumor area in a mouse).

### Example 15. Evaluation of the tumor therapeutic effect of a combination of IL12 + GMCSF

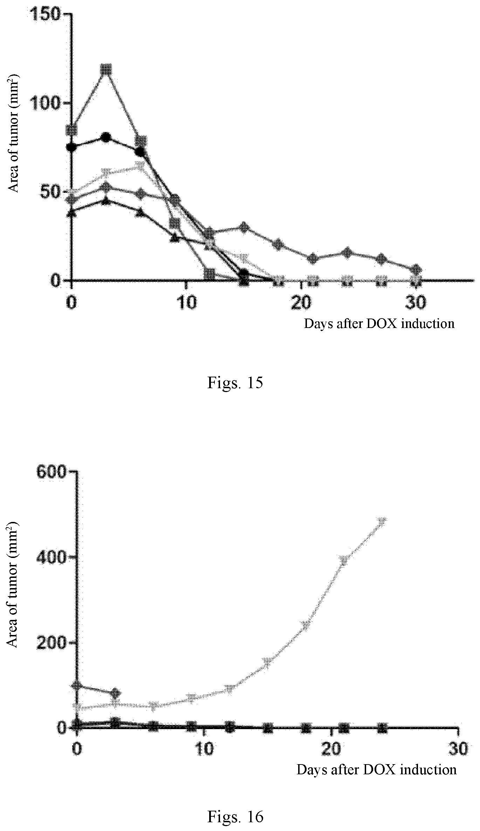

B16 (rtTA) -IL12 and B16 (rtTA) -GMCSF cells in the logarithmic growth phase were digested, and resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (15 mice in total). The mice were fed with water containing 2 g/L of doxycycline after the tumor grew. The mouse tumor growth and tumor clearance were recorded, and number of mice with tumor cleared/total number of mice is equal to 13/15. The results show that the combination of IL12 + GMCSF can induce the tumor regression in some tested mice (Fig. 15, wherein each broken line represents the in vivo tumor area in a mouse).

### Example 16. Evaluation of the tumor therapeutic effect of a combination of IFNγ + IL2 + LT

B16 (rtTA) -IFNγ, B16 (rtTA) -IL2 and B16 (rtTA) -LT cells in the logarithmic growth phase were digested, and resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (12 mice in total). The mice were fed with water containing 2 g/L of doxycycline after the tumor grew. The mouse tumor growth and tumor clearance were recorded, and number of mice with tumor cleared/total number of mice is equal to 7/12. The results show that the combination of IFNγ + IL2 + LT can induce the tumor regression in some tested mice (Fig. 16, wherein each broken line represents the in vivo tumor area in a mouse).

### Example 17. Construction of cells comprising the second expression vectors encoding FMS-associated tyrosine kinase 3 ligand (FLT3L), interleukin 7 (IL7)

FLT3L (GenBank: NM_013520) and IL7 (GenBank: NM_008371) gene encoding regions were synthesized with BamHI and EcoRI restriction sites at both ends of each gene, respectively, and then digested by BamHI and EcoRI with the following digestion system: 5µg of plasmid with the gene, 4 µl of digestion buffer, 1 µl of BamHI, 1 µl of EcoRI. The mixture was supplemented with water to a total volume of 40 µl, and stood at 37°C for 12 hr. 4.4 µl 10x loading buffer was added into the EP tube and an electrophoresis was performed with 1% agarose gel, and after the electrophoresis, the gene fragments were recovered until usage. The regulating expression vector pLentis-PTRE-MCS-PGK-PURO was digested with the following digestion system: 2µg of plasmid, 3 µl of digestion buffer, 1 µl of BamHI, 1 µl of EcoRI. The mixture was supplemented with water to a total volume of 30 µl, and stood at 37°C for 12 hr. 3.3 µl 10x loading buffer was added into the EP tube and an electrophoresis was performed with 1% agarose gel, and after the electrophoresis, the vector fragments were recovered until usage. pLentis-PTRE-MCS-PGK-PURO and the target gene were ligated with the following ligation system: 2 µl of pLentis-PTRE-MCS-PGK-PURO, 2 µl of target gene, 1 µl of ligase buffer, 0.5 µl of T4DNAligase, 4.5 µl of water. The mixture was ligated at room temperature for 4 hr. Then, the linking system was subject to the transformation of *E. coli* competent cells. At the next day, colonies were picked from the transformed plates, and cultured in an LB media at 37°C in a shaker overnight. Plasmids were extracted from the cultured cells with a plasmid extract kit, digested for identifying if the fragments were successfully ligated into the vector, and then the right vector was sent for sequencing, to determine the successful construction of the second expression vector pLentis-PTRE-FLT3L-PGK-PURO, and pLentis-PTRE-IL7-PGK-PURO. The expression vector virus was prepared by the same method as that of preparing the first expression vector virus. The cultured B16 (rtTA) tumor cells were digested, and seeded at 10⁵ cells/well into a 6-well plate, and the culture volume was 1 ml. After 24 hr, 10 µl of the vector virus for regulating the expression was added, and cultured in an incubator for additional 24 hr. The supernatant was discarded, and replaced with fresh media to continue culture. After the cells grow over the bottom, they were transferred to a culture flask, and puromycin was added to a final concentration of 3 µg/ml. After being cultured for additional 3 days, the surviving cells were those capable of regulating the expression of FLT3L and IL7, respectively, which were in turn named B16 (rtTA) -FLT3L and B16 (rtTA) -IL7.

### Example 18. Evaluation of the tumor therapeutic effect of a combination of IL12 + GMCSF + IL7

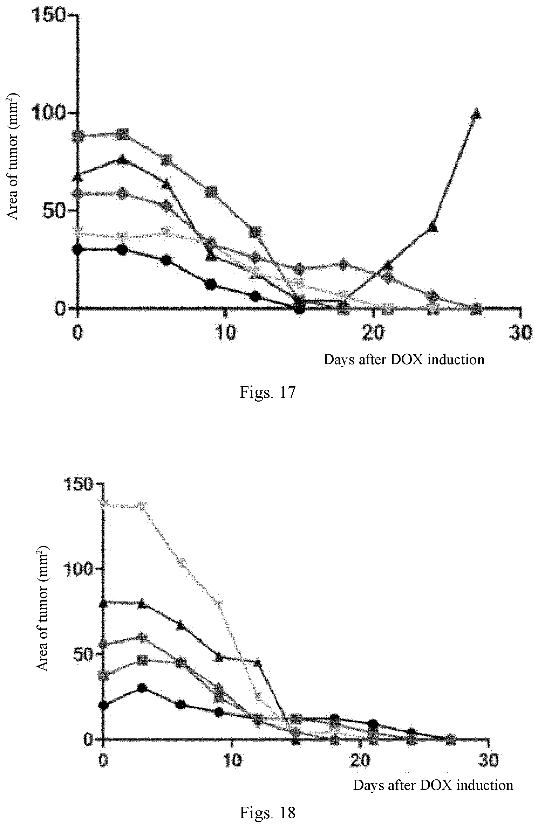

B16 (rtTA) -IL12, B16 (rtTA) -GMCSF and B16 (rtTA) -IL7 cells in the logarithmic growth phase were digested, and resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (15 mice in total). The mice were fed with water containing 2 g/L of doxycycline after the tumor grew. The mouse tumor growth and tumor clearance were recorded, and number of mice with tumor cleared/total number of mice is equal to 13/15. The results show that the combination of IL12 + GMCSF + IL7 can induce the tumor regression in some tested mice (Fig. 17, wherein each broken line represents the in vivo tumor area in a mouse).

### Example 19. Evaluation of the tumor therapeutic effect of a combination of IL12 + FLT3L + IL7

B16 (rtTA) -IL12, B16 (rtTA) -FLT3L and B16 (rtTA) -IL7 cells in the logarithmic growth phase were digested, and the two types of cells were resuspended in HBSS to form a mixed solution, wherein the concentrations of each type of cells was 1 x 10⁶ cells/ml. The mixed solution was injected at a dose of 50 µl with a 1 ml injector to the right dorsal side of a C57BL/6 female mouse at 8-10 weeks (15 mice in total). The mice were fed with water containing 2 g/L of doxycycline after the tumor grew. The mouse tumor growth and tumor clearance were recorded, and number of mice with tumor cleared/total number of mice is equal to 13/15. The results show that the combination of IL12 + FLT3L + IL7 can induce the tumor regression in some tested mice (Fig. 18, wherein each broken line represents the in vivo tumor area in a mouse).

The foregoing detailed description is provided by way of explanation and example, and is not intended to limit the scope of the appended claims. Many variations of the embodiments as listed herein are obvious to those of ordinary skill in the art, and are within the scope of the appended claims and their equivalents.

SEQUENCE LISTING

<110>  张晋宇

<120>  用于筛选抗肿瘤物质的系统及方法

<130>  0069-PA-004

<160>  3

<170>  PatentIn version 3.5

<210>  1
<211>  7964
<212>  DNA
<213>  人工序列(Artificial Sequence)

<220>
<223>  pLentis-CMV-rtTA-IRES-Bsd

<400>  1
acgcgtgtag tcttatgcaa tactcttgta gtcttgcaac atggtaacga tgagttagca      60

acatgcctta caaggagaga aaaagcaccg tgcatgccga ttggtggaag taaggtggta     120

cgatcgtgcc ttattaggaa ggcaacagac gggtctgaca tggattggac gaaccactga     180

attgccgcat tgcagagata ttgtatttaa gtgcctagct cgatacaata aacgggtctc     240

tctggttaga ccagatctga gcctgggagc tctctggcta actagggaac ccactgctta     300

agcctcaata aagcttgcct tgagtgcttc aagtagtgtg tgcccgtctg ttgtgtgact     360

ctggtaacta gagatccctc agaccctttt agtcagtgtg gaaaatctct agcagtggcg     420

cccgaacagg gacctgaaag cgaaagggaa accagagctc tctcgacgca ggactcggct     480

tgctgaagcg cgcacggcaa gaggcgaggg gcggcgactg gtgagtacgc caaaaatttt     540

gactagcgga ggctagaagg agagagatgg gtgcgagagc gtcagtatta agcgggggag     600

aattagatcg cgatgggaaa aaattcggtt aaggccaggg ggaaagaaaa aatataaatt     660

aaaacatata gtatgggcaa gcagggagct agaacgattc gcagttaatc ctggcctgtt     720

agaaacatca gaaggctgta gacaaatact gggacagcta caaccatccc ttcagacagg     780

atcagaagaa cttagatcat tatataatac agtagcaacc ctctattgtg tgcatcaaag     840

gatagagata aaagacacca aggaagcttt agacaagata gaggaagagc aaaacaaaag     900

taagaccacc gcacagcaag cggccactga tcttcagacc tggaggagga gatatgaggg     960

acaattggag aagtgaatta tataaatata aagtagtaaa aattgaacca ttaggagtag    1020

cacccaccaa ggcaaagaga agagtggtgc agagagaaaa aagagcagtg ggaataggag    1080

ctttgttcct tgggttcttg ggagcagcag gaagcactat gggcgcagcc tcaatgacgc    1140

tgacggtaca ggccagacaa ttattgtctg gtatagtgca gcagcagaac aatttgctga 1200

gggctattga ggcgcaacag catctgttgc aactcacagt ctggggcatc aagcagctcc 1260

aggcaagaat cctggctgtg gaaagatacc taaaggatca acagctcctg gggatttggg 1320

gttgctctgg aaaactcatt tgcaccactg ctgtgccttg gaatgctagt tggagtaata 1380

aatctctgga acagattgga atcacacgac ctggatggag tgggacagag aaattaacaa 1440

ttacacaagc ttaatacact ccttaattga agaatcgcaa aaccagcaag aaaagaatga 1500

acaagaatta ttggaattag ataaatgggc aagtttgtgg aattggttta acataacaaa 1560

ttggctgtgg tatataaaat tattcataat gatagtagga ggcttggtag gtttaagaat 1620

agtttttgct gtactttcta tagtgaatag agttaggcag ggatattcac cattatcgtt 1680

tcagacccac ctcccaaccc cgaggggacc cgacaggccc gaaggaatag aagaagaagg 1740

tggagagaga gacagagaca gatccattcg attagtgaac ggatctcgac ggttaacttt 1800

taaaagaaaa ggggggattg gggggtacag tgcaggggaa agaatagtag acataatagc 1860

aacagacata caaactaaag aattacaaaa acaaattaca aaaattcaaa attttatcga 1920

tactgcaggt actagtatta tgcccagtac atgaccttat gggactttcc tacttggcag 1980

tacatctacg tattagtcat cgctattacc atggtgatgc ggttttggca gtacatcaat 2040

gggcgtggat agcggtttga ctcacgggga tttccaagtc tccaccccat tgacgtcaat 2100

gggagtttgt tttggcacca aaatcaacgg gactttccaa aatgtcgtaa caactccgcc 2160

ccattgacgc aaatgggcgg taggcgtgta cggtgggagg tctatataag cagagctcgt 2220

ttagtgaacc gtcagatcgc ctggagacgc catccacgct gttttgacct ccatagaaga 2280

ggatccaagc caccatgtct agactggaca agagcaaagt cataaacgga gctctggaat 2340

tactcaatgg tgtcggtatc gaaggcctga cgacaaggaa actcgctcaa aagctgggag 2400

ttgagcagcc taccctgtac tggcacgtga agaacaagcg ggccctgctc gatgccctgc 2460

caatcgagat gctggacagg catcataccc acttctgccc cctggaaggc gagtcatggc 2520

aagactttct gcggaacaac gccaagtcat accgctgtgc tctcctctca catcgcgacg 2580

gggctaaagt gcatctcggc acccgcccaa cagagaaaca gtacgaaacc ctggaaaatc 2640

agctcgcgtt cctgtgtcag caaggcttct ccctggagaa cgcactgtac gctctgtccg 2700

ccgtgggcca ctttacactg ggctgcgtat tggaggaaca ggagcatcaa gtagcaaaag 2760

aggaaagaga gacacctacc accgattcta tgcccccact tctgagacaa gcaattgagc 2820

tgttcgaccg gcagggagcc gaacctgcct tccttttcgg cctggaacta atcatatgtg 2880

gcctggagaa acagctaaag tgcgaaagcg gcgggccgac cgacgccctt gacgattttg 2940

acttagacat gctcccagcc gatgcccttg acgactttga ccttgatatg ctgcctgctg 3000

acgctcttga cgattttgac cttgacatgc tccccgggta agtcgagagt gctagcgccc 3060

ctctccctcc ccccccccta acgttactgg ccgaagccgc ttggaataag gccggtgtgc 3120

gtttgtctat atgttatttt ccaccatatt gccgtctttt ggcaatgtga gggcccggaa 3180

acctggccct gtcttcttga cgagcattcc taggggtctt tcccctctcg ccaaaggaat 3240

gcaaggtctg ttgaatgtcg tgaaggaagc agttcctctg gaagcttctt gaagacaaac 3300

aacgtctgta gcgacccttt gcaggcagcg gaacccccca cctggcgaca ggtgcctctg 3360

cggccaaaag ccacgtgtat aagatacacc tgcaaaggcg gcacaacccc agtgccacgt 3420

tgtgagttgg atagttgtgg aaagagtcaa atggctctcc tcaagcgtat tcaacaaggg 3480

gctgaaggat gcccagaagg taccccattg tatgggatct gatctggggc ctcggtacac 3540

atgctttaca tgtgtttagt cgaggttaaa aaaacgtcta ggccccccga accacgggga 3600

cgtggttttc ctttgaaaaa cacgatgata atatggccac agaattctag aatggttcct 3660

ttgtctcaag aagaatccac cctcattgaa agagcaacgg ctacaatcaa cagcatcccc 3720

atctctgaag actacagcgt cgccagcgca gctctctcta gcgacggccg catcttcact 3780

ggtgtcaatg tatatcattt tactggggga ccttgtgcag aactcgtggt gctgggcact 3840

gctgctgctg cggcagctgg caacctgact tgtatcgtcg cgatcggaaa tgagaacagg 3900

ggcatcttga gcccctgcgg acggtgccga caggtgcttc tcgatctgca tcctgggatc 3960

aaagccatag tgaaggacag tgatggacag ccgacggcag ttgggattcg tgaattgctg 4020

ccctctggtt atgtgtggga gggctaagtc gacatcgaga atcaacctct ggattacaaa 4080

atttgtgaaa gattgactgg tattcttaac tatgttgctc cttttacgct atgtggatac 4140

gctgctttaa tgcctttgta tcatgctatt gcttcccgta tggctttcat tttctcctcc 4200

ttgtataaat cctggttgct gtctctttat gaggagttgt ggcccgttgt caggcaacgt 4260

ggcgtggtgt gcactgtgtt tgctgacgca acccccactg gttggggcat tgccaccacc 4320

tgtcagctcc tttccgggac tttcgctttc cccctcccta ttgccacggc ggaactcatc 4380

gccgcctgcc ttgcccgctg ctggacaggg gctcggctgt tgggcactga caattccgtg 4440

gtgttgtcgg ggaagctgac gtcctttcca tggctgctcg cctgtgttgc cacctggatt 4500

ctgcgcggga cgtccttctg ctacgtccct tcggccctca atccagcgga ccttccttcc 4560

cgcggcctgc tgccggctct gcggcctctt ccgcgtctcc gccttcgccc tcagacgagt 4620

cggatctccc tttggccgcc tccccgcctg gtacctttaa gaccaatgac ttacaaggca 4680

gctgtagatc ttagccactt tttaaaagaa aaggggggac tggaagggct aattcactcc 4740

caacgaaaat aagatctgct ttttgcttgt actgggtctc tctggttaga ccagatctga 4800

gcctgggagc tctctggcta actagggaac ccactgctta agcctcaata aagcttgcct 4860

tgagtgcttc aagtagtgtg tgcccgtctg ttgtgtgact ctggtaacta gagatccctc 4920

agaccctttt agtcagtgtg gaaaatctct agcagtagta gttcatgtca tcttattatt 4980

cagtatttat aacttgcaaa gaaatgaata tcagagagtg agaggaactt gtttattgca 5040

gcttataatg gttacaaata aagcaatagc atcacaaatt tcacaaataa agcatttttt 5100

tcactgcatt ctagttgtgg tttgtccaaa ctcatcaatg tatcttatca tgtctggctc 5160

tagctatccc gcccctaact ccgcccagtt ccgcccattc tccgccccat ggctgactaa 5220

ttttttttat ttatgcagag gccgaggccg cctcggcctc tgagctattc cagaagtagt 5280

gaggaggctt ttttggaggc ctagactttt gcagagacgg cccaaattcg taatcatggt 5340

catagctgtt tcctgtgtga aattgttatc cgctcacaat tccacacaac atacgagccg 5400

gaagcataaa gtgtaaagcc tggggtgcct aatgagtgag ctaactcaca ttaattgcgt 5460

tgcgctcact gcccgctttc cagtcgggaa acctgtcgtg ccagctgcat taatgaatcg 5520

gccaacgcgc ggggagaggc ggtttgcgta ttgggcgctc ttccgcttcc tcgctcactg 5580

actcgctgcg ctcggtcgtt cggctgcggc gagcggtatc agctcactca aaggcggtaa 5640

tacggttatc cacagaatca ggggataacg caggaaagaa catgtgagca aaaggccagc 5700

aaaaggccag gaaccgtaaa aaggccgcgt tgctggcgtt tttccatagg ctccgccccc 5760

ctgacgagca tcacaaaaat cgacgctcaa gtcagaggtg gcgaaacccg acaggactat 5820

aaagatacca ggcgtttccc cctggaagct ccctcgtgcg ctctcctgtt ccgaccctgc 5880

cgcttaccgg atacctgtcc gcctttctcc cttcgggaag cgtggcgctt tctcatagct 5940

cacgctgtag gtatctcagt tcggtgtagg tcgttcgctc caagctgggc tgtgtgcacg 6000

aaccccccgt tcagcccgac cgctgcgcct tatccggtaa ctatcgtctt gagtccaacc 6060

cggtaagaca cgacttatcg ccactggcag cagccactgg taacaggatt agcagagcga 6120

ggtatgtagg cggtgctaca gagttcttga agtggtggcc taactacggc tacactagaa 6180

ggacagtatt tggtatctgc gctctgctga agccagttac cttcggaaaa agagttggta 6240

gctcttgatc cggcaaacaa accaccgctg gtagcggtgg tttttttgtt tgcaagcagc 6300

agattacgcg cagaaaaaaa ggatctcaag aagatccttt gatcttttct acggggtctg 6360

acgctcagtg gaacgaaaac tcacgttaag ggattttggt catgagatta tcaaaaagga 6420

tcttcaccta gatcctttta aattaaaaat gaagttttaa atcaatctaa agtatatatg 6480

agtaaacttg gtctgacagt taccaatgct taatcagtga ggcacctatc tcagcgatct 6540

gtctatttcg ttcatccata gttgcctgac tccccgtcgt gtagataact acgatacggg 6600

agggcttacc atctggcccc agtgctgcaa tgataccgcg agacccacgc tcaccggctc 6660

cagatttatc agcaataaac cagccagccg gaagggccga gcgcagaagt ggtcctgcaa 6720

ctttatccgc ctccatccag tctattaatt gttgccggga agctagagta agtagttcgc 6780

cagttaatag tttgcgcaac gttgttgcca ttgctacagg catcgtggtg tcacgctcgt 6840

cgtttggtat ggcttcattc agctccggtt cccaacgatc aaggcgagtt acatgatccc 6900

ccatgttgtg caaaaaagcg gttagctcct tcggtcctcc gatcgttgtc agaagtaagt 6960

tggccgcagt gttatcactc atggttatgg cagcactgca taattctctt actgtcatgc 7020

catccgtaag atgcttttct gtgactggtg agtactcaac caagtcattc tgagaatagt 7080

gtatgcggcg accgagttgc tcttgcccgg cgtcaatacg ggataatacc gcgccacata 7140

gcagaacttt aaaagtgctc atcattggaa aacgttcttc ggggcgaaaa ctctcaagga 7200

tcttaccgct gttgagatcc agttcgatgt aacccactcg tgcacccaac tgatcttcag 7260

catcttttac tttcaccagc gtttctgggt gagcaaaaac aggaaggcaa aatgccgcaa 7320

aaaagggaat aagggcgaca cggaaatgtt gaatactcat actcttcctt tttcaatatt 7380

attgaagcat ttatcagggt tattgtctca tgagcggata catatttgaa tgtatttaga 7440

aaaataaaca aataggggtt ccgcgcacat ttccccgaaa agtgccacct gacgtctaag 7500

aaaccattat tatcatgaca ttaacctata aaaataggcg tatcacgagg ccctttcgtc 7560

tcgcgcgttt cggtgatgac ggtgaaaacc tctgacacat gcagctcccg gagacggtca 7620

cagcttgtct gtaagcggat gccgggagca gacaagcccg tcagggcgcg tcagcgggtg 7680

ttggcgggtg tcggggctgg cttaactatg cggcatcaga gcagattgta ctgagagtgc 7740

accatatgcg gtgtgaaata ccgcacagat gcgtaaggag aaaataccgc atcaggcgcc 7800

attcgccatt caggctgcgc aactgttggg aagggcgatc ggtgcgggcc tcttcgctat 7860

tacgccagct ggcgaaaggg ggatgtgctg caaggcgatt aagttgggta acgccagggt 7920

tttcccagtc acgacgttgt aaaacgacgg ccagtgccaa gctg 7964

<210> 2
<211> 7322

<212>  DNA
<213>  人工序列(Artificial Sequence)

<220>
<223>  pLentis-PTRE-MCS-PGK-PURO

<400>  2
acgcgtgtag tcttatgcaa tactcttgta gtcttgcaac atggtaacga tgagttagca      60

acatgcctta caaggagaga aaaagcaccg tgcatgccga ttggtggaag taaggtggta     120

cgatcgtgcc ttattaggaa ggcaacagac gggtctgaca tggattggac gaaccactga     180

attgccgcat tgcagagata ttgtatttaa gtgcctagct cgatacaata aacgggtctc     240

tctggttaga ccagatctga gcctgggagc tctctggcta actagggaac ccactgctta     300

agcctcaata aagcttgcct tgagtgcttc aagtagtgtg tgcccgtctg ttgtgtgact     360

ctggtaacta gagatccctc agaccctttt agtcagtgtg gaaaatctct agcagtggcg     420

cccgaacagg gacctgaaag cgaaagggaa accagagctc tctcgacgca ggactcggct     480

tgctgaagcg cgcacggcaa gaggcgaggg gcggcgactg gtgagtacgc caaaaatttt     540

gactagcgga ggctagaagg agagagatgg gtgcgagagc gtcagtatta agcgggggag     600

aattagatcg cgatgggaaa aaattcggtt aaggccaggg ggaaagaaaa aatataaatt     660

aaaacatata gtatgggcaa gcagggagct agaacgattc gcagttaatc ctggcctgtt     720

agaaacatca gaaggctgta gacaaatact gggacagcta caaccatccc ttcagacagg     780

atcagaagaa cttagatcat tatataatac agtagcaacc ctctattgtg tgcatcaaag     840

gatagagata aaagacacca aggaagcttt agacaagata gaggaagagc aaaacaaaag     900

taagaccacc gcacagcaag cggccactga tcttcagacc tggaggagga gatatgaggg     960

acaattggag aagtgaatta tataaatata aagtagtaaa aattgaacca ttaggagtag    1020

cacccaccaa ggcaaagaga agagtggtgc agagagaaaa aagagcagtg ggaataggag    1080

ctttgttcct tgggttcttg ggagcagcag gaagcactat gggcgcagcc tcaatgacgc    1140

tgacggtaca ggccagacaa ttattgtctg gtatagtgca gcagcagaac aatttgctga    1200

gggctattga ggcgcaacag catctgttgc aactcacagt ctggggcatc aagcagctcc    1260

aggcaagaat cctggctgtg gaaagatacc taaaggatca acagctcctg gggatttggg    1320

gttgctctgg aaaactcatt tgcaccactg ctgtgccttg gaatgctagt tggagtaata    1380

aatctctgga acagattgga atcacacgac ctggatggag tgggacagag aaattaacaa    1440

ttacacaagc ttaatacact ccttaattga agaatcgcaa aaccagcaag aaaagaatga    1500

acaagaatta ttggaattag ataaatgggc aagtttgtgg aattggttta acataacaaa    1560

ttggctgtgg tatataaaat tattcataat gatagtagga ggcttggtag gtttaagaat 1620

agtttttgct gtactttcta tagtgaatag agttaggcag ggatattcac cattatcgtt 1680

tcagacccac ctcccaaccc cgaggggacc cgacaggccc gaaggaatag aagaagaagg 1740

tggagagaga gacagagaca gatccattcg attagtgaac ggatctcgac ggttaacttt 1800

taaaagaaaa ggggggattg gggggtacag tgcaggggaa agaatagtag acataatagc 1860

aacagacata caaactaaag aattacaaaa acaaattaca aaaattcaaa attttatcga 1920

tgagtttact ccctatcagt gatagagaac gtatgtcgag tttactccct atcagtgata 1980

gagaacgatg tcgagtttac tccctatcag tgatagagaa cgtatgtcga gtttactccc 2040

tatcagtgat agagaacgta tgtcgagttt actccctatc agtgatagag aacgtatgtc 2100

gagtttatcc ctatcagtga tagagaacgt atgtcgagtt tactccctat cagtgataga 2160

gaacgtatgt cgaggtaggc gtgtacggtg ggaggcctat ataagcagag ctcgtttagt 2220

gaaccgtcag atcgccggat ccgctagcaa ggatatcaac tgtacaagtg aattcatatc 2280

tcgaggggta ggggaggcgc ttttcccaag gcagtctgga gcatgcgctt tagcagcccc 2340

gctgggcact tggcgctaca caagtggcct ctggcctcgc acacattcca catccaccgg 2400

taggcgccaa ccggctccgt tctttggtgg ccccttcgcg ccaccttcta ctcctcccct 2460

agtcaggaag ttcccccccg ccccgcagct cgcgtcgtgc aggacgtgac aaatggaagt 2520

agcacgtctc actagtctcg tgcagatgga cagcaccgct gagcaatgga agcgggtagg 2580

cctttggggc agcggccaat agcagctttg ctccttcgct ttctgggctc agaggctggg 2640

aaggggtggg tccgggggcg ggctcagggg cgggctcagg ggcggggcgg gcgcccgaag 2700

gtcctccgga ggcccggcat tctgcacgct tcaaaagcgc acgtctgccg cgctgttctc 2760

ctcttcctca tctccgggcc tttcgacctg cagcccaagt ctagaatgac cgagtacaag 2820

cccacggtgc gcctcgccac ccgcgacgac gtccccaggg ccgtacgcac cctcgccgcc 2880

gcgttcgccg actaccccgc cacgcgccac accgtcgatc cggaccgcca catcgagcgg 2940

gtcaccgagc tgcaagaact cttcctcacg cgcgtcgggc tcgacatcgg caaggtgtgg 3000

gtcgcggacg acggcgccgc ggtggcggtc tggaccacgc cggagagcgt cgaagcgggg 3060

gcggtgttcg ccgagatcgg cccgcgcatg gccgagttga gcggttcccg gctggccgcg 3120

cagcaacaga tggaaggcct cctggcgccg caccggccca aggagcccgc gtggttcctg 3180

gccaccgtcg gcgtctcgcc cgaccaccag ggcaagggtc tgggcagcgc cgtcgtgctc 3240

cccggagtgg aggcggccga gcgcgccggg gtgcccgcct tcctggagac ctccgcgccc 3300

cgcaacctcc ccttctacga gcggctcggc ttcaccgtca ccgccgacgt cgaggtgccc 3360

gaaggaccgc gcacctggtg catgacccgc aagcccggtg cctgagtcga catcgagaat 3420

caacctctgg attacaaaat ttgtgaaaga ttgactggta ttcttaacta tgttgctcct 3480

tttacgctat gtggatacgc tgctttaatg cctttgtatc atgctattgc ttcccgtatg 3540

gctttcattt tctcctcctt gtataaatcc tggttgctgt ctctttatga ggagttgtgg 3600

cccgttgtca ggcaacgtgg cgtggtgtgc actgtgtttg ctgacgcaac ccccactggt 3660

tggggcattg ccaccacctg tcagctcctt tccgggactt tcgctttccc cctccctatt 3720

gccacggcgg aactcatcgc cgcctgcctt gcccgctgct ggacaggggc tcggctgttg 3780

ggcactgaca attccgtggt gttgtcgggg aagctgacgt cctttccatg gctgctcgcc 3840

tgtgttgcca cctggattct gcgcgggacg tccttctgct acgtcccttc ggccctcaat 3900

ccagcggacc ttccttcccg cggcctgctg ccggctctgc ggcctcttcc gcgtctccgc 3960

cttcgccctc agacgagtcg gatctccctt tggccgcctc cccgcctggt acctttaaga 4020

ccaatgactt acaaggcagc tgtagatctt agccactttt taaaagaaaa ggggggactg 4080

gaagggctaa ttcactccca acgaaaataa gatctgcttt ttgcttgtac tgggtctctc 4140

tggttagacc agatctgagc ctgggagctc tctggctaac tagggaaccc actgcttaag 4200

cctcaataaa gcttgccttg agtgcttcaa gtagtgtgtg cccgtctgtt gtgtgactct 4260

ggtaactaga gatccctcag acccttttag tcagtgtgga aaatctctag cagtagtagt 4320

tcatgtcatc ttattattca gtatttataa cttgcaaaga aatgaatatc agagagtgag 4380

aggaacttgt ttattgcagc ttataatggt tacaaataaa gcaatagcat cacaaatttc 4440

acaaataaag catttttttc actgcattct agttgtggtt tgtccaaact catcaatgta 4500

tcttatcatg tctggctcta gctatcccgc ccctaactcc gcccagttcc gcccattctc 4560

cgccccatgg ctgactaatt ttttttattt atgcagaggc cgaggccgcc tcggcctctg 4620

agctattcca gaagtagtga ggaggctttt ttggaggcct agacttttgc agagacggcc 4680

caaattcgta atcatggtca tagctgtttc ctgtgtgaaa ttgttatccg ctcacaattc 4740

cacacaacat acgagccgga agcataaagt gtaaagcctg gggtgcctaa tgagtgagct 4800

aactcacatt aattgcgttg cgctcactgc ccgctttcca gtcgggaaac ctgtcgtgcc 4860

agctgcatta atgaatcggc caacgcgcgg ggagaggcgg tttgcgtatt gggcgctctt 4920

ccgcttcctc gctcactgac tcgctgcgct cggtcgttcg gctgcggcga gcggtatcag 4980

ctcactcaaa ggcggtaata cggttatcca cagaatcagg ggataacgca ggaaagaaca 5040

tgtgagcaaa aggccagcaa aaggccagga accgtaaaaa ggccgcgttg ctggcgtttt 5100

tccataggct ccgcccccct gacgagcatc acaaaaatcg acgctcaagt cagaggtggc 5160

gaaacccgac aggactataa agataccagg cgtttccccc tggaagctcc ctcgtgcgct 5220

ctcctgttcc gaccctgccg cttaccggat acctgtccgc ctttctccct tcgggaagcg 5280

tggcgctttc tcatagctca cgctgtaggt atctcagttc ggtgtaggtc gttcgctcca 5340

agctgggctg tgtgcacgaa ccccccgttc agcccgaccg ctgcgcctta tccggtaact 5400

atcgtcttga gtccaacccg gtaagacacg acttatcgcc actggcagca gccactggta 5460

acaggattag cagagcgagg tatgtaggcg gtgctacaga gttcttgaag tggtggccta 5520

actacggcta cactagaagg acagtatttg gtatctgcgc tctgctgaag ccagttacct 5580

tcggaaaaag agttggtagc tcttgatccg gcaaacaaac caccgctggt agcggtggtt 5640

tttttgtttg caagcagcag attacgcgca gaaaaaaagg atctcaagaa gatcctttga 5700

tcttttctac ggggtctgac gctcagtgga acgaaaactc acgttaaggg attttggtca 5760

tgagattatc aaaaaggatc ttcacctaga tccttttaaa ttaaaaatga agttttaaat 5820

caatctaaag tatatatgag taaacttggt ctgacagtta ccaatgctta atcagtgagg 5880

cacctatctc agcgatctgt ctatttcgtt catccatagt tgcctgactc cccgtcgtgt 5940

agataactac gatacgggag ggcttaccat ctggccccag tgctgcaatg ataccgcgag 6000

acccacgctc accggctcca gatttatcag caataaacca gccagccgga agggccgagc 6060

gcagaagtgg tcctgcaact ttatccgcct ccatccagtc tattaattgt tgccgggaag 6120

ctagagtaag tagttcgcca gttaatagtt tgcgcaacgt tgttgccatt gctacaggca 6180

tcgtggtgtc acgctcgtcg tttggtatgg cttcattcag ctccggttcc caacgatcaa 6240

ggcgagttac atgatccccc atgttgtgca aaaaagcggt tagctccttc ggtcctccga 6300

tcgttgtcag aagtaagttg gccgcagtgt tatcactcat ggttatggca gcactgcata 6360

attctcttac tgtcatgcca tccgtaagat gcttttctgt gactggtgag tactcaacca 6420

agtcattctg agaatagtgt atgcggcgac cgagttgctc ttgcccggcg tcaatacggg 6480

ataataccgc gccacatagc agaactttaa aagtgctcat cattggaaaa cgttcttcgg 6540

ggcgaaaact ctcaaggatc ttaccgctgt tgagatccag ttcgatgtaa cccactcgtg 6600

cacccaactg atcttcagca tcttttactt tcaccagcgt ttctgggtga gcaaaaacag 6660

gaaggcaaaa tgccgcaaaa aagggaataa gggcgacacg gaaatgttga atactcatac 6720

tcttcctttt tcaatattat tgaagcattt atcagggtta ttgtctcatg agcggataca 6780

tatttgaatg tatttagaaa aataaacaaa taggggttcc gcgcacattt ccccgaaaag 6840

tgccacctga cgtctaagaa accattatta tcatgacatt aacctataaa aataggcgta 6900

tcacgaggcc ctttcgtctc gcgcgtttcg gtgatgacgg tgaaaacctc tgacacatgc 6960

agctcccgga gacggtcaca gcttgtctgt aagcggatgc cgggagcaga caagcccgtc 7020

agggcgcgtc agcgggtgtt ggcgggtgtc ggggctggct taactatgcg gcatcagagc 7080

agattgtact gagagtgcac catatgcggt gtgaaatacc gcacagatgc gtaaggagaa 7140

aataccgcat caggcgccat tcgccattca ggctgcgcaa ctgttgggaa gggcgatcgg 7200

tgcgggcctc ttcgctatta cgccagctgg cgaaaggggg atgtgctgca aggcgattaa 7260

gttgggtaac gccagggttt tcccagtcac gacgttgtaa aacgacggcc agtgccaagc 7320

tg 7322

<210> 3
<211> 248
<212> PRT
<213> 人工序列(Artificial Sequence)

<220>
<223> rtTA

<400> 3

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Tyr Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Thr Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

Page 1 of 10

SEQUENCE LISTING

<110>  ZHANG, Jinyu

<120>  SYSTEM AND METHOD FOR SCREENING ANTI-TUMOR SUBSTANCES

<130>  0069-PA-004EP

<160>  3

<170>  PatentIn version 3.5

<210>  1
<211>  7964
<212>  DNA
<213>  Artificial Sequence

<220>
<223>  pLentis-CMV-rtTA-IRES-Bsd

<400>  1
acgcgtgtag tcttatgcaa tactcttgta gtcttgcaac atggtaacga tgagttagca      60

acatgcctta caaggagaga aaaagcaccg tgcatgccga ttggtggaag taaggtggta     120

cgatcgtgcc ttattaggaa ggcaacagac gggtctgaca tggattggac gaaccactga     180

attgccgcat tgcagagata ttgtatttaa gtgcctagct cgatacaata aacgggtctc     240

tctggttaga ccagatctga gcctgggagc tctctggcta actagggaac ccactgctta     300

agcctcaata aagcttgcct tgagtgcttc aagtagtgtg tgcccgtctg ttgtgtgact     360

ctggtaacta gagatccctc agaccctttt agtcagtgtg gaaaatctct agcagtggcg     420

cccgaacagg gacctgaaag cgaaagggaa accagagctc tctcgacgca ggactcggct     480

tgctgaagcg cgcacggcaa gaggcgaggg gcggcgactg gtgagtacgc caaaaatttt     540

gactagcgga ggctagaagg agagagatgg gtgcgagagc gtcagtatta agcgggggag     600

aattagatcg cgatgggaaa aaattcggtt aaggccaggg ggaaagaaaa aatataaatt     660

aaaacatata gtatgggcaa gcagggagct agaacgattc gcagttaatc ctggcctgtt     720

agaaacatca gaaggctgta gacaaatact gggacagcta caaccatccc ttcagacagg     780

atcagaagaa cttagatcat tatataatac agtagcaacc ctctattgtg tgcatcaaag     840

gatagagata aaagacacca aggaagcttt agacaagata gaggaagagc aaaacaaaag     900

taagaccacc gcacagcaag cggccactga tcttcagacc tggaggagga gatatgaggg     960

acaattggag aagtgaatta tataaatata aagtagtaaa aattgaacca ttaggagtag    1020

cacccaccaa ggcaaagaga agagtggtgc agagagaaaa aagagcagtg ggaataggag    1080

ctttgttcct tgggttcttg ggagcagcag gaagcactat gggcgcagcc tcaatgacgc    1140

tgacggtaca ggccagacaa ttattgtctg gtatagtgca gcagcagaac aatttgctga    1200

gggctattga ggcgcaacag catctgttgc aactcacagt ctggggcatc aagcagctcc    1260

Page 2 of 10

aggcaagaat cctggctgtg gaaagatacc taaaggatca acagctcctg gggatttggg 1320

gttgctctgg aaaactcatt tgcaccactg ctgtgccttg gaatgctagt tggagtaata 1380

aatctctgga acagattgga atcacacgac ctggatggag tgggacagag aaattaacaa 1440

ttacacaagc ttaatacact ccttaattga agaatcgcaa aaccagcaag aaaagaatga 1500

acaagaatta ttggaattag ataaatgggc aagtttgtgg aattggttta acataacaaa 1560

ttggctgtgg tatataaaat tattcataat gatagtagga ggcttggtag gtttaagaat 1620

agtttttgct gtactttcta tagtgaatag agttaggcag ggatattcac cattatcgtt 1680

tcagacccac ctcccaaccc cgaggggacc cgacaggccc gaaggaatag aagaagaagg 1740

tggagagaga gacagagaca gatccattcg attagtgaac ggatctcgac ggttaacttt 1800

taaaagaaaa ggggggattg gggggtacag tgcaggggaa agaatagtag acataatagc 1860

aacagacata caaactaaag aattacaaaa acaaattaca aaaattcaaa attttatcga 1920

tactgcaggt actagtatta tgcccagtac atgaccttat gggactttcc tacttggcag 1980

tacatctacg tattagtcat cgctattacc atggtgatgc ggttttggca gtacatcaat 2040

gggcgtggat agcggtttga ctcacgggga tttccaagtc tccaccccat tgacgtcaat 2100

gggagtttgt tttggcacca aaatcaacgg gactttccaa aatgtcgtaa caactccgcc 2160

ccattgacgc aaatgggcgg taggcgtgta cggtgggagg tctatataag cagagctcgt 2220

ttagtgaacc gtcagatcgc ctggagacgc catccacgct gttttgacct ccatagaaga 2280

ggatccaagc caccatgtct agactggaca agagcaaagt cataaacgga gctctggaat 2340

tactcaatgg tgtcggtatc gaaggcctga cgacaaggaa actcgctcaa aagctgggag 2400

ttgagcagcc taccctgtac tggcacgtga agaacaagcg ggccctgctc gatgccctgc 2460

caatcgagat gctggacagg catcataccc acttctgccc cctggaaggc gagtcatggc 2520

aagactttct gcggaacaac gccaagtcat accgctgtgc tctcctctca catcgcgacg 2580

gggctaaagt gcatctcggc acccgcccaa cagagaaaca gtacgaaacc ctggaaaatc 2640

agctcgcgtt cctgtgtcag caaggcttct ccctggagaa cgcactgtac gctctgtccg 2700

ccgtgggcca ctttacactg ggctgcgtat tggaggaaca ggagcatcaa gtagcaaaag 2760

aggaaagaga gacacctacc accgattcta tgcccccact tctgagacaa gcaattgagc 2820

tgttcgaccg gcagggagcc gaacctgcct tccttttcgg cctggaacta atcatatgtg 2880

gcctggagaa acagctaaag tgcgaaagcg gcgggccgac cgacgccctt gacgattttg 2940

acttagacat gctcccagcc gatgcccttg acgactttga ccttgatatg ctgcctgctg 3000

acgctcttga cgattttgac cttgacatgc tccccgggta agtcgagagt gctagcgccc 3060

ctctccctcc ccccccccta acgttactgg ccgaagccgc ttggaataag gccggtgtgc 3120

gtttgtctat atgttatttt ccaccatatt gccgtctttt ggcaatgtga gggcccggaa 3180

Page 3 of 10

acctggccct gtcttcttga cgagcattcc taggggtctt tcccctctcg ccaaaggaat 3240

gcaaggtctg ttgaatgtcg tgaaggaagc agttcctctg gaagcttctt gaagacaaac 3300

aacgtctgta gcgacccttt gcaggcagcg gaacccccca cctggcgaca ggtgcctctg 3360

cggccaaaag ccacgtgtat aagatacacc tgcaaaggcg gcacaacccc agtgccacgt 3420

tgtgagttgg atagttgtgg aaagagtcaa atggctctcc tcaagcgtat tcaacaaggg 3480

gctgaaggat gcccagaagg taccccattg tatgggatct gatctggggc ctcggtacac 3540

atgctttaca tgtgtttagt cgaggttaaa aaaacgtcta ggccccccga accacgggga 3600

cgtggttttc ctttgaaaaa cacgatgata atatggccac agaattctag aatggttcct 3660

ttgtctcaag aagaatccac cctcattgaa agagcaacgg ctacaatcaa cagcatcccc 3720

atctctgaag actacagcgt cgccagcgca gctctctcta gcgacggccg catcttcact 3780

ggtgtcaatg tatatcattt tactggggga ccttgtgcag aactcgtggt gctgggcact 3840

gctgctgctg cggcagctgg caacctgact tgtatcgtcg cgatcggaaa tgagaacagg 3900

ggcatcttga gcccctgcgg acggtgccga caggtgcttc tcgatctgca tcctgggatc 3960

aaagccatag tgaaggacag tgatggacag ccgacggcag ttgggattcg tgaattgctg 4020

ccctctggtt atgtgtggga gggctaagtc gacatcgaga atcaacctct ggattacaaa 4080

atttgtgaaa gattgactgg tattcttaac tatgttgctc cttttacgct atgtggatac 4140

gctgctttaa tgcctttgta tcatgctatt gcttcccgta tggctttcat tttctcctcc 4200

ttgtataaat cctggttgct gtctctttat gaggagttgt ggcccgttgt caggcaacgt 4260

ggcgtggtgt gcactgtgtt tgctgacgca acccccactg gttggggcat tgccaccacc 4320

tgtcagctcc tttccgggac tttcgctttc cccctcccta ttgccacggc ggaactcatc 4380

gccgcctgcc ttgcccgctg ctggacaggg gctcggctgt tgggcactga caattccgtg 4440

gtgttgtcgg ggaagctgac gtcctttcca tggctgctcg cctgtgttgc cacctggatt 4500

ctgcgcggga cgtccttctg ctacgtccct tcggccctca atccagcgga ccttccttcc 4560

cgcggcctgc tgccggctct gcggcctctt ccgcgtctcc gccttcgccc tcagacgagt 4620

cggatctccc tttggccgcc tccccgcctg gtacctttaa gaccaatgac ttacaaggca 4680

gctgtagatc ttagccactt tttaaaagaa aaggggggac tggaagggct aattcactcc 4740

caacgaaaat aagatctgct ttttgcttgt actgggtctc tctggttaga ccagatctga 4800

gcctgggagc tctctggcta actagggaac ccactgctta agcctcaata aagcttgcct 4860

tgagtgcttc aagtagtgtg tgcccgtctg ttgtgtgact ctggtaacta gagatccctc 4920

agaccctttt agtcagtgtg gaaaatctct agcagtagta gttcatgtca tcttattatt 4980

cagtatttat aacttgcaaa gaaatgaata tcagagagtg agaggaactt gtttattgca 5040

Page 4 of 10

gcttataatg gttacaaata aagcaatagc atcacaaatt tcacaaataa agcatttttt 5100

tcactgcatt ctagttgtgg tttgtccaaa ctcatcaatg tatcttatca tgtctggctc 5160

tagctatccc gcccctaact ccgcccagtt ccgcccattc tccgccccat ggctgactaa 5220

ttttttttat ttatgcagag gccgaggccg cctcggcctc tgagctattc cagaagtagt 5280

gaggaggctt ttttggaggc ctagactttt gcagagacgg cccaaattcg taatcatggt 5340

catagctgtt tcctgtgtga aattgttatc cgctcacaat tccacacaac atacgagccg 5400

gaagcataaa gtgtaaagcc tggggtgcct aatgagtgag ctaactcaca ttaattgcgt 5460

tgcgctcact gcccgctttc cagtcgggaa acctgtcgtg ccagctgcat taatgaatcg 5520

gccaacgcgc ggggagaggc ggtttgcgta ttgggcgctc ttccgcttcc tcgctcactg 5580

actcgctgcg ctcggtcgtt cggctgcggc gagcggtatc agctcactca aaggcggtaa 5640

tacggttatc cacagaatca ggggataacg caggaaagaa catgtgagca aaaggccagc 5700

aaaaggccag gaaccgtaaa aaggccgcgt tgctggcgtt tttccatagg ctccgccccc 5760

ctgacgagca tcacaaaaat cgacgctcaa gtcagaggtg gcgaaacccg acaggactat 5820

aaagatacca ggcgtttccc cctggaagct ccctcgtgcg ctctcctgtt ccgaccctgc 5880

cgcttaccgg atacctgtcc gcctttctcc cttcgggaag cgtggcgctt tctcatagct 5940

cacgctgtag gtatctcagt tcggtgtagg tcgttcgctc caagctgggc tgtgtgcacg 6000

aaccccccgt tcagcccgac cgctgcgcct tatccggtaa ctatcgtctt gagtccaacc 6060

cggtaagaca cgacttatcg ccactggcag cagccactgg taacaggatt agcagagcga 6120

ggtatgtagg cggtgctaca gagttcttga agtggtggcc taactacggc tacactagaa 6180

ggacagtatt tggtatctgc gctctgctga agccagttac cttcggaaaa agagttggta 6240

gctcttgatc cggcaaacaa accaccgctg gtagcggtgg tttttttgtt tgcaagcagc 6300

agattacgcg cagaaaaaaa ggatctcaag aagatccttt gatcttttct acggggtctg 6360

acgctcagtg gaacgaaaac tcacgttaag ggattttggt catgagatta tcaaaaagga 6420

tcttcaccta gatcctttta aattaaaaat gaagttttaa atcaatctaa agtatatatg 6480

agtaaacttg gtctgacagt taccaatgct taatcagtga ggcacctatc tcagcgatct 6540

gtctatttcg ttcatccata gttgcctgac tccccgtcgt gtagataact acgatacggg 6600

agggcttacc atctggcccc agtgctgcaa tgataccgcg agacccacgc tcaccggctc 6660

cagatttatc agcaataaac cagccagccg gaagggccga gcgcagaagt ggtcctgcaa 6720

ctttatccgc ctccatccag tctattaatt gttgccggga agctagagta agtagttcgc 6780

cagttaatag tttgcgcaac gttgttgcca ttgctacagg catcgtggtg tcacgctcgt 6840

cgtttggtat ggcttcattc agctccggtt cccaacgatc aaggcgagtt acatgatccc 6900

ccatgttgtg caaaaaagcg gttagctcct tcggtcctcc gatcgttgtc agaagtaagt 6960

Page 5 of 10

tggccgcagt gttatcactc atggttatgg cagcactgca taattctctt actgtcatgc 7020

catccgtaag atgcttttct gtgactggtg agtactcaac caagtcattc tgagaatagt 7080

gtatgcggcg accgagttgc tcttgcccgg cgtcaatacg ggataatacc gcgccacata 7140

gcagaacttt aaaagtgctc atcattggaa aacgttcttc ggggcgaaaa ctctcaagga 7200

tcttaccgct gttgagatcc agttcgatgt aacccactcg tgcacccaac tgatcttcag 7260

catcttttac tttcaccagc gtttctgggt gagcaaaaac aggaaggcaa aatgccgcaa 7320

aaaagggaat aagggcgaca cggaaatgtt gaatactcat actcttcctt tttcaatatt 7380

attgaagcat ttatcagggt tattgtctca tgagcggata catatttgaa tgtatttaga 7440

aaaataaaca aataggggtt ccgcgcacat ttccccgaaa agtgccacct gacgtctaag 7500

aaaccattat tatcatgaca ttaacctata aaaataggcg tatcacgagg ccctttcgtc 7560

tcgcgcgttt cggtgatgac ggtgaaaacc tctgacacat gcagctcccg gagacggtca 7620

cagcttgtct gtaagcggat gccgggagca gacaagcccg tcagggcgcg tcagcgggtg 7680

ttggcgggtg tcggggctgg cttaactatg cggcatcaga gcagattgta ctgagagtgc 7740

accatatgcg gtgtgaaata ccgcacagat gcgtaaggag aaaataccgc atcaggcgcc 7800

attcgccatt caggctgcgc aactgttggg aagggcgatc ggtgcgggcc tcttcgctat 7860

tacgccagct ggcgaaaggg ggatgtgctg caaggcgatt aagttgggta acgccagggt 7920

tttcccagtc acgacgttgt aaaacgacgg ccagtgccaa gctg 7964

<210> 2
<211> 7322
<212> DNA
<213> Artificial Sequence

<220>
<223> pLentis-PTRE-MCS-PGK-PURO

<400> 2
acgcgtgtag tcttatgcaa tactcttgta gtcttgcaac atggtaacga tgagttagca 60

acatgcctta caaggagaga aaaagcaccg tgcatgccga ttggtggaag taaggtggta 120

cgatcgtgcc ttattaggaa ggcaacagac gggtctgaca tggattggac gaaccactga 180

attgccgcat tgcagagata ttgtatttaa gtgcctagct cgatacaata aacgggtctc 240

tctggttaga ccagatctga gcctgggagc tctctggcta actagggaac ccactgctta 300

agcctcaata aagcttgcct tgagtgcttc aagtagtgtg tgcccgtctg ttgtgtgact 360

ctggtaacta gagatccctc agaccctttt agtcagtgtg gaaaatctct agcagtggcg 420

cccgaacagg gacctgaaag cgaaagggaa accagagctc tctcgacgca ggactcggct 480

tgctgaagcg cgcacggcaa gaggcgaggg gcggcgactg gtgagtacgc caaaaatttt 540

Page 6 of 10

gactagcgga ggctagaagg agagagatgg gtgcgagagc gtcagtatta agcgggggag 600

aattagatcg cgatgggaaa aaattcggtt aaggccaggg ggaaagaaaa aatataaatt 660

aaaacatata gtatgggcaa gcagggagct agaacgattc gcagttaatc ctggcctgtt 720

agaaacatca gaaggctgta gacaaatact gggacagcta caaccatccc ttcagacagg 780

atcagaagaa cttagatcat tatataatac agtagcaacc ctctattgtg tgcatcaaag 840

gatagagata aaagacacca aggaagcttt agacaagata gaggaagagc aaaacaaaag 900

taagaccacc gcacagcaag cggccactga tcttcagacc tggaggagga gatatgaggg 960

acaattggag aagtgaatta tataaatata aagtagtaaa aattgaacca ttaggagtag 1020

cacccaccaa ggcaaagaga agagtggtgc agagagaaaa aagagcagtg ggaataggag 1080

ctttgttcct tgggttcttg ggagcagcag gaagcactat gggcgcagcc tcaatgacgc 1140

tgacggtaca ggccagacaa ttattgtctg gtatagtgca gcagcagaac aatttgctga 1200

gggctattga ggcgcaacag catctgttgc aactcacagt ctggggcatc aagcagctcc 1260

aggcaagaat cctggctgtg gaaagatacc taaaggatca acagctcctg gggatttggg 1320

gttgctctgg aaaactcatt tgcaccactg ctgtgccttg gaatgctagt tggagtaata 1380

aatctctgga acagattgga atcacacgac ctggatggag tgggacagag aaattaacaa 1440

ttacacaagc ttaatacact ccttaattga agaatcgcaa aaccagcaag aaaagaatga 1500

acaagaatta ttggaattag ataaatgggc aagtttgtgg aattggttta acataacaaa 1560

ttggctgtgg tatataaaat tattcataat gatagtagga ggcttggtag gtttaagaat 1620

agtttttgct gtactttcta tagtgaatag agttaggcag ggatattcac cattatcgtt 1680

tcagacccac ctcccaaccc cgaggggacc cgacaggccc gaaggaatag aagaagaagg 1740

tggagagaga gacagagaca gatccattcg attagtgaac ggatctcgac ggttaacttt 1800

taaaagaaaa ggggggattg gggggtacag tgcaggggaa agaatagtag acataatagc 1860

aacagacata caaactaaag aattacaaaa acaaattaca aaaattcaaa attttatcga 1920

tgagtttact ccctatcagt gatagagaac gtatgtcgag tttactccct atcagtgata 1980

gagaacgatg tcgagtttac tccctatcag tgatagagaa cgtatgtcga gtttactccc 2040

tatcagtgat agagaacgta tgtcgagttt actccctatc agtgatagag aacgtatgtc 2100

gagtttatcc ctatcagtga tagagaacgt atgtcgagtt tactccctat cagtgataga 2160

gaacgtatgt cgaggtaggc gtgtacggtg ggaggcctat ataagcagag ctcgtttagt 2220

gaaccgtcag atcgccggat ccgctagcaa ggatatcaac tgtacaagtg aattcatatc 2280

tcgaggggta ggggaggcgc ttttcccaag gcagtctgga gcatgcgctt tagcagcccc 2340

gctgggcact tggcgctaca caagtggcct ctggcctcgc acacattcca catccaccgg 2400

taggcgccaa ccggctccgt tctttggtgg ccccttcgcg ccaccttcta ctcctcccct 2460

Page 7 of 10

agtcaggaag ttcccccccg ccccgcagct cgcgtcgtgc aggacgtgac aaatggaagt 2520

agcacgtctc actagtctcg tgcagatgga cagcaccgct gagcaatgga agcgggtagg 2580

cctttggggc agcggccaat agcagctttg ctccttcgct ttctgggctc agaggctggg 2640

aaggggtggg tccgggggcg ggctcagggg cgggctcagg ggcggggcgg gcgcccgaag 2700

gtcctccgga ggcccggcat tctgcacgct tcaaaagcgc acgtctgccg cgctgttctc 2760

ctcttcctca tctccgggcc tttcgacctg cagcccaagt ctagaatgac cgagtacaag 2820

cccacggtgc gcctcgccac ccgcgacgac gtccccaggg ccgtacgcac cctcgccgcc 2880

gcgttcgccg actaccccgc cacgcgccac accgtcgatc cggaccgcca catcgagcgg 2940

gtcaccgagc tgcaagaact cttcctcacg cgcgtcgggc tcgacatcgg caaggtgtgg 3000

gtcgcggacg acggcgccgc ggtggcggtc tggaccacgc cggagagcgt cgaagcgggg 3060

gcggtgttcg ccgagatcgg cccgcgcatg gccgagttga gcggttcccg gctggccgcg 3120

cagcaacaga tggaaggcct cctggcgccg caccggccca aggagcccgc gtggttcctg 3180

gccaccgtcg gcgtctcgcc cgaccaccag ggcaagggtc tgggcagcgc cgtcgtgctc 3240

cccggagtgg aggcggccga gcgcgccggg gtgcccgcct tcctggagac ctccgcgccc 3300

cgcaacctcc ccttctacga gcggctcggc ttcaccgtca ccgccgacgt cgaggtgccc 3360

gaaggaccgc gcacctggtg catgacccgc aagcccggtg cctgagtcga catcgagaat 3420

caacctctgg attacaaaat ttgtgaaaga ttgactggta ttcttaacta tgttgctcct 3480

tttacgctat gtggatacgc tgctttaatg cctttgtatc atgctattgc ttcccgtatg 3540

gctttcattt tctcctcctt gtataaatcc tggttgctgt ctctttatga ggagttgtgg 3600

cccgttgtca ggcaacgtgg cgtggtgtgc actgtgtttg ctgacgcaac ccccactggt 3660

tggggcattg ccaccacctg tcagctcctt tccgggactt tcgctttccc cctccctatt 3720

gccacggcgg aactcatcgc cgcctgcctt gcccgctgct ggacaggggc tcggctgttg 3780

ggcactgaca attccgtggt gttgtcgggg aagctgacgt cctttccatg gctgctcgcc 3840

tgtgttgcca cctggattct gcgcgggacg tccttctgct acgtcccttc ggccctcaat 3900

ccagcggacc ttccttcccg cggcctgctg ccggctctgc ggcctcttcc gcgtctccgc 3960

cttcgccctc agacgagtcg gatctccctt tggccgcctc cccgcctggt acctttaaga 4020

ccaatgactt acaaggcagc tgtagatctt agccactttt taaaagaaaa ggggggactg 4080

gaagggctaa ttcactccca acgaaaataa gatctgcttt ttgcttgtac tgggtctctc 4140

tggttagacc agatctgagc ctgggagctc tctggctaac tagggaaccc actgcttaag 4200

cctcaataaa gcttgccttg agtgcttcaa gtagtgtgtg cccgtctgtt gtgtgactct 4260

ggtaactaga gatccctcag acccttttag tcagtgtgga aaatctctag cagtagtagt 4320

Page 8 of 10

tcatgtcatc ttattattca gtatttataa cttgcaaaga aatgaatatc agagagtgag 4380

aggaacttgt ttattgcagc ttataatggt tacaaataaa gcaatagcat cacaaatttc 4440

acaaataaag catttttttc actgcattct agttgtggtt tgtccaaact catcaatgta 4500

tcttatcatg tctggctcta gctatcccgc ccctaactcc gcccagttcc gcccattctc 4560

cgccccatgg ctgactaatt ttttttattt atgcagaggc cgaggccgcc tcggcctctg 4620

agctattcca gaagtagtga ggaggctttt ttggaggcct agacttttgc agagacggcc 4680

caaattcgta atcatggtca tagctgtttc ctgtgtgaaa ttgttatccg ctcacaattc 4740

cacacaacat acgagccgga agcataaagt gtaaagcctg gggtgcctaa tgagtgagct 4800

aactcacatt aattgcgttg cgctcactgc ccgctttcca gtcgggaaac ctgtcgtgcc 4860

agctgcatta atgaatcggc caacgcgcgg ggagaggcgg tttgcgtatt gggcgctctt 4920

ccgcttcctc gctcactgac tcgctgcgct cggtcgttcg gctgcggcga gcggtatcag 4980

ctcactcaaa ggcggtaata cggttatcca cagaatcagg ggataacgca ggaaagaaca 5040

tgtgagcaaa aggccagcaa aaggccagga accgtaaaaa ggccgcgttg ctggcgtttt 5100

tccataggct ccgcccccct gacgagcatc acaaaaatcg acgctcaagt cagaggtggc 5160

gaaacccgac aggactataa agataccagg cgtttccccc tggaagctcc ctcgtgcgct 5220

ctcctgttcc gaccctgccg cttaccggat acctgtccgc ctttctccct tcgggaagcg 5280

tggcgctttc tcatagctca cgctgtaggt atctcagttc ggtgtaggtc gttcgctcca 5340

agctgggctg tgtgcacgaa ccccccgttc agcccgaccg ctgcgcctta tccggtaact 5400

atcgtcttga gtccaacccg gtaagacacg acttatcgcc actggcagca gccactggta 5460

acaggattag cagagcgagg tatgtaggcg gtgctacaga gttcttgaag tggtggccta 5520

actacggcta cactagaagg acagtatttg gtatctgcgc tctgctgaag ccagttacct 5580

tcggaaaaag agttggtagc tcttgatccg gcaaacaaac caccgctggt agcggtggtt 5640

tttttgtttg caagcagcag attacgcgca gaaaaaaagg atctcaagaa gatcctttga 5700

tcttttctac ggggtctgac gctcagtgga acgaaaactc acgttaaggg attttggtca 5760

tgagattatc aaaaaggatc ttcacctaga tccttttaaa ttaaaaatga agttttaaat 5820

caatctaaag tatatatgag taaacttggt ctgacagtta ccaatgctta atcagtgagg 5880

cacctatctc agcgatctgt ctatttcgtt catccatagt tgcctgactc cccgtcgtgt 5940

agataactac gatacgggag ggcttaccat ctggccccag tgctgcaatg ataccgcgag 6000

acccacgctc accggctcca gatttatcag caataaacca gccagccgga agggccgagc 6060

gcagaagtgg tcctgcaact ttatccgcct ccatccagtc tattaattgt tgccgggaag 6120

ctagagtaag tagttcgcca gttaatagtt tgcgcaacgt tgttgccatt gctacaggca 6180

tcgtggtgtc acgctcgtcg tttggtatgg cttcattcag ctccggttcc caacgatcaa 6240

Page 9 of 10

ggcgagttac atgatccccc atgttgtgca aaaaagcggt tagctccttc ggtcctccga 6300

tcgttgtcag aagtaagttg gccgcagtgt tatcactcat ggttatggca gcactgcata 6360

attctcttac tgtcatgcca tccgtaagat gcttttctgt gactggtgag tactcaacca 6420

agtcattctg agaatagtgt atgcggcgac cgagttgctc ttgcccggcg tcaatacggg 6480

ataataccgc gccacatagc agaactttaa aagtgctcat cattggaaaa cgttcttcgg 6540

ggcgaaaact ctcaaggatc ttaccgctgt tgagatccag ttcgatgtaa cccactcgtg 6600

cacccaactg atcttcagca tcttttactt tcaccagcgt ttctgggtga gcaaaaacag 6660

gaaggcaaaa tgccgcaaaa aagggaataa gggcgacacg gaaatgttga atactcatac 6720

tcttcctttt tcaatattat tgaagcattt atcagggtta ttgtctcatg agcggataca 6780

tatttgaatg tatttagaaa aataaacaaa taggggttcc gcgcacattt ccccgaaaag 6840

tgccacctga cgtctaagaa accattatta tcatgacatt aacctataaa aataggcgta 6900

tcacgaggcc ctttcgtctc gcgcgtttcg gtgatgacgg tgaaaacctc tgacacatgc 6960

agctcccgga gacggtcaca gcttgtctgt aagcggatgc cgggagcaga caagcccgtc 7020

agggcgcgtc agcgggtgtt ggcgggtgtc ggggctggct taactatgcg gcatcagagc 7080

agattgtact gagagtgcac catatgcggt gtgaaatacc gcacagatgc gtaaggagaa 7140

aataccgcat caggcgccat tcgccattca ggctgcgcaa ctgttgggaa gggcgatcgg 7200

tgcgggcctc ttcgctatta cgccagctgg cgaaaggggg atgtgctgca aggcgattaa 7260

gttgggtaac gccagggttt tcccagtcac gacgttgtaa aacgacggcc agtgccaagc 7320

tg 7322

<210> 3
<211> 248
<212> PRT
<213> Artificial Sequence

<220>
<223> rtTA

<400> 3

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His

Page 10 of 10
50                  55                  60

Thr His Phe Cys Pro Leu Glu Gly Glu Ser Trp Gln Asp Phe Leu Arg
65                  70                  75                  80

Asn Asn Ala Lys Ser Tyr Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Thr Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

## Claims

1. A system for screening anti-tumor substances, comprising:
1) a first expression vector encoding a first regulatory factor; and
2) a second expression vector encoding a biological macromolecule, wherein an expression of the biological macromolecule can be co-regulated by the first regulatory factor expressed by the first expression vector and an inducer, and the biological macromolecule is a candidate anti-tumor substance to be screened.

2. The system according to claim 1, wherein the first regulatory factor and the inducer co-activate the expression of the biological macromolecule encoded by the second expression vector.

3. The system according to any one of claims 1-2, wherein the system comprises two or more types of second expression vectors, and the biological macromolecule encoded by one type of second expression vector is different from the biological macromolecule encoded by at least another type of second expression vector in the system.

4. The system according to any one of claims 1-3, wherein the system comprises a cell, and the cell contains the first expression vector and/or the second expression vector.

5. The system according to claim 4, wherein the system comprises two or more types of cells, each type of cells comprises the first expression vector and the second expression vector, and the biological macromolecule encoded by the second expression vector in one type of cells is different from the biological macromolecule encoded by the second expression vector in at least another type of cells in the system.

6. The system according to any one of claims 4-5, wherein the cell comprises a tumor cell.

7. The system according to any one of claims 1-6, wherein the first expression vector comprises a first promoter, the first promoter is operably linked to a sequence encoding the first regulatory factor, and the first promoter is a constitutive promoter.

8. The system according to any one of claims 1-7, wherein the second expression vector comprises a second promoter, the second promoter is an inducible promoter which can be co-activated by the first regulatory factor and the inducer, and the second promoter is operably linked to a sequence encoding the biological macromolecule.

9. Use of the system according to any one of claims 1-8 for preparation of a kit for screening anti-tumor substances.

10. A method for screening anti-tumor substances, comprising:
1) providing a cell comprising a first expression vector encoding a first regulatory factor and a second expression vector encoding a biological macromolecule, wherein an expression of the biological macromolecule can be co-regulated by the first regulatory factor expressed by the first expression vector and an inducer;
2) administering the inducer, so that the inducer and the first regulatory factor co-activate the expression of the biological macromolecule encoded by the second expression vector; and
3) detecting the effect of the expression of the biological macromolecule on the growth and/or survival of the tumor, wherein the biological macromolecule is a candidate anti-tumor substance to be screened.

11. The method according to claim 10, wherein the cell comprising the first expression vector and the second expression vector comprises two or more types of cells, each type of cells comprises the first expression vector and the second expression vector, and the biological macromolecule encoded by the second expression vector in each type of cells is different from the biological macromolecule encoded by the second expression vector in at least another type of cells.

12. An anti-tumor substance screened by the method according to any one of claims 10-11, comprising a combination selected from the group consisting of:
1) IPNγ, IL2 and LT;
2) IL12, GMCSF and IL7; and
3) IL12, FLT3L and IL7.

13. A method for preparing the system according to any one of claims 1-11, comprising:
1) constructing the first expression vector; and
2) constructing the second expression vector.

14. The method according to claim 13, further comprising:
3) introducing the first expression vector and the second expression vector into two or more types of cells, wherein each type of cells comprises the first expression vector and the second expression vector, and the biological macromolecule encoded by the second expression vector in each type of cells is different from the biological macromolecule encoded by the second expression vector in at least another type of cells.
